# EUROPEAN PATENT SPECIFICATION

(11) **EP 0 863 768 B1**
(45) Date of publication and mention of the grant of the patent: **03.01.2007**
(21) Application number: 96939779.3
(22) Date of filing: 29.11.1996
(51) Int. Cl.: A61K 47/48, A61P 11/00, A61P 7/02, A61L 27/34

(54) **Covalent heparin-protein conjugates comprising an alpha-carbonyl amine bond and methods of obtaining them**
Heparine-proteine kovalente Konjugate, die eine Alpha-Carbonyl-Amin enthalten und Verfahren für deren Produktion
Conjugués covalents de l'héparine et protéine comprenant une liaison alpha-carbonyl -amine et des méthodes pour leur obtention

(30) Priority: 30.11.1995 US 564976
(43) Date of publication of application: 16.09.1998
(73) Proprietor: McMASTER UNIVERSITY, Hamilton, Ontario L8S 4L8 (CA); Hamilton Health Sciences Corporation, Ontario, L8S 1B7 (CA)
(72) Inventor: BERRY, Leslie, Burlington, Ontario L7S 1N8 (CA); ANDREW, Maureen, Oakville, Ontario L6H 3A8 (CA)
(74) Representative: Holliday, Louise Caroline
(86) International application number: PCT/CA1996/000799
(87) International publication number: WO 1997/019701

(56) References cited:
- EP-A- 0 098 814
- EP-A- 0 137 356
- FR-A- 2 635 019
- US-A- 3 842 061
- US-A- 4 689 323
- JOURNAL OF BIOLOGICAL CHEMISTRY, vol. 265, no. 30, 25 October 1990, MD US, pages 18263-18271, XP002034213 M.M. MAIMONE ET AL.: "STRUCTURE OF A DERMATAN SULFATE HEXASACCHARIDE THAT BINDS TO HEPARIN COFACTOR II WITH HIGH AFFINTY." cited in the application
- CHEMICAL ABSTRACTS, vol. 122, no. 25, 19 June 1995 Columbus, Ohio, US; abstract no. 306176, UNO, TAKEJI ET AL: "Evaluation for antithrombogenicity on the surface of heparinized biomedical material using thrombin-antithrombin III complex" XP002034214 & SEITAI ZAIRYO (1995), 13(1), 21-5 CODEN: SEZAEH;ISSN: 0910-304X, 1995,
- THROMB. RES. (1978), 13(4), 655-70 CODEN: THBRAA;ISSN: 0049-3848, 1978, XP000676774 HATTON, M. W. C. ET AL: "Inhibition of thrombin by antithrombin III in the presence of certain glycosaminoglycans found in the mammalian aorta"
- J. CARDIOVASC. PHARMACOL., vol. 7, no. SUPPL.3, 1985, pages S191-S205, XP000676772 VERSTRAETE M.: "PREVENTION OF THROMBOSIS IN ARTERIES: NOVEL APPROACHES."

## Description

### BACKGROUND OF THE INVENTION

### 1. Field of the Invention

This invention relates to new chemical compounds comprising covalent conjugates of glycosaminoglycans, particularly heparins, methods for their preparation, their pharmaceutical compositions and therapeutic uses thereof.

### 2. Description of the Background Art

Heparin is a sulfated polysaccharide which consists largely of an alternating sequence of hexuronic acid and 2-amino-2-deoxy-D-glucose. Heparin and a related compound, dermatan sulfate, are of great importance as anticoagulants for clinical use in the prevention of thrombosis and related diseases. They are members of the family of glycosaminoglycans, (GAGs), which are linear chains of sulfated repeating disaccharide units containing a hexosamine and a uronic acid. Anticoagulation using GAGs (such as heparin and dermatan sulfate) proceeds via their catalysis of inhibition of coagulant enzymes (the critical one being thrombin) by serine protease inhibitors (serpins) such as antithrombin III (ATIII) and heparin cofactor II (HCII). Binding of the serpins by the catalysts is critical for their action and occurs through specific sequences along the linear carbohydrate chain of the glycosaminoglycan (GAG). Heparin acts by binding to ATIII via a pentasaccharide sequence, thus potentiating inhibition of a variety of coagulant enzymes (in the case of thrombin, heparin must also bind to the enzyme). Heparin can also potentiate inhibition of thrombin by binding to the serpin HCII. Dermatan sulfate acts by specifically binding to HCII via a hexasaccharide sequence, thus potentiating only the inhibition of thrombin. Since glycosaminoglycans (particularly heparin) can bind to other molecules *in vivo* or be lost from the site of action due to a variety of mechanisms, it would be advantageous to keep the GAG permanently associated with the serpin by a covalent bond.

Covalent complexes between ATIII and heparin have been produced previously; see, *e.g.,* Bjork et al., (1982) *FEBS Letters* 143(1):96-100, and by Collen et al., U.S. Patent No. 4,623,718. These conjugates required covalent modification of the heparin prior to its conjugation. The product by Bjork et al. (produced by reduction of the Schiff base between the aldehyde of a 2,5-D-anhydromannose terminus of heparin, produced by partial depolymerization of heparin to heparin fragments with nitrous acid, and a lysyl amino of ATIII) had undetectable antithrombin activity. The product by Collen et al. (produced by conjugation of carboxyl groups within the chain of the heparin molecule and lysyl amino groups of ATIII through amino-hexyl tolyl spacer arms) had a random attachment to the carboxyls of the uronic acids of the heparin moiety that might affect the ATIII binding sequence and in fact the specific anti-Xa (a coagulation protease which activates prothrombin to thrombin) activity was approximately 65% of the starting non-covalently linked unmodified heparin (*J. Biol. Chem.* 257:3401-3408 (1982)). The specific anti-thrombin activity would also be, therefore, 65 % or less since both Xa and thrombin require heparin binding to ATIII. The bimolecular rate constant of the product by Collen et al. for inhibition of thrombin was claimed to be comparable to that of noncovalent mixtures of heparin saturated with ATIII (*J. Biol. Chem.* 259:5670-5677 (1984)). However, large molar excesses of heparin or covalent complex over thrombin (> 10: 1) were used to simplify the kinetics, which would mask the effect of any subpopulation of molecules with low activity. Specific antithrombin activities were not given.

In addition, heparin has also been covalently conjugated to other proteins (such as tissue plasminogen activator and erythropoietin) by Halluin (U.S. Patent No. 5,308,617), using a similar method to that of Bjork et al. These conjugates suffered from the same problems associated with loss of heparin activity as with the Bjork conjugates. Coupling of heparin to affinity supports via a hydrazine linkage is reported in WO 95/05400. However, the hydrazine group is not commonly found in proteins and other macromolecules, and its incorporation often results in a decrease in biological activity. U.S. Patent No. 4,213,962 describes heparin and antithrombin III coimmobilized on cyanogen bromide activated agarose. U.S. Patents Nos. 5,280,016 and 4,990,502 describe the oxidation of heparin with periodate and reduction of the aldehydes so generated.

Therefore, it would be desirable to provide covalent conjugates of heparin and related glycosaminoglycans which retain maximal biological activity (*e.g.*, anticoagulant activity) and improved pharmacokinetic properties and simple methods for their preparation. This invention fulfills these and other needs.

### SUMMARY OR THE INVENTION

This invention provides a covalent conjugate comprising a glycosaminoglycan linked to a protein by a covalent linkage wherein the protein comprises at least one primary amino group, wherein the protein is directly covalently linked via its amino group to a terminal aldose residue of the glycosaminoglycan.

The covalent linkage comprises an α-carbonyl amine formed by subsequent Amadori rearrangement of an imine (> C=N-) formed between the amino group of the first species and the C1 of the terminal aldose. The glycosaminoglycan is preferably heparin. The amine containing species is a protein such as antithrombin III or heparin cofactor II.

The invention also provides novel and mild methods of preparing the above covalent conjugates which result in conjugates with improved pharmacokinetic properties and biological activity. The methods comprise incubating the glycosaminoglycans with the amine-containing protein under conditions which allow imine formation between the terminal aldose residue of the glycosaminoglycan and the amine. The imine is allowed to rearrange under mild conditions (Amadori rearrangement) to an α-carbonyl amine. The invention further provides pharmaceutical compositions comprising these conjugates and therapeutic uses thereof.

The invention also provides methods for reducing the thrombogenicity of a material, such as a synthetic polymer, by coating the material with the covalent conjugates of the inventions, especially the heparin-antithrombin III conjugate. Materials treated by this method are useful as medical or prosthetic devices.

### BRIEF DESCRIPTION OF THE DRAWINGS

Figure 1 compares the inhibition of thrombin activity by antithrombin III, noncovalent antithrombin III-heparin complexes and various concentrations of the covalent antithrombin III-heparin (ATH) conjugates of the present invention.
Figure 2 shows the inhibition of the ability of thrombin to clot human fibrinogen by the covalent antithrombin III-heparin conjugates (ATH1) of the present invention.
Figure 3 shows the effect of added heparin on the rate of inhibition of thrombin by the antithrombin III-heparin conjugates of the present invention.
Figure 4 shows the rate of inhibition of thrombin activity towards the chromogenic substrate S-2238 by the antithrombin III-heparin conjugates of the present invention.
Figure 5 shows the inhibition of the antithrombin effect of the covalent ATH conjugates of the present invention by FPR-thrombin.
Figure 6 shows the plasma clearance of the covalent ATH conjugates of the present invention and heparin in rabbits after intravenous injection.

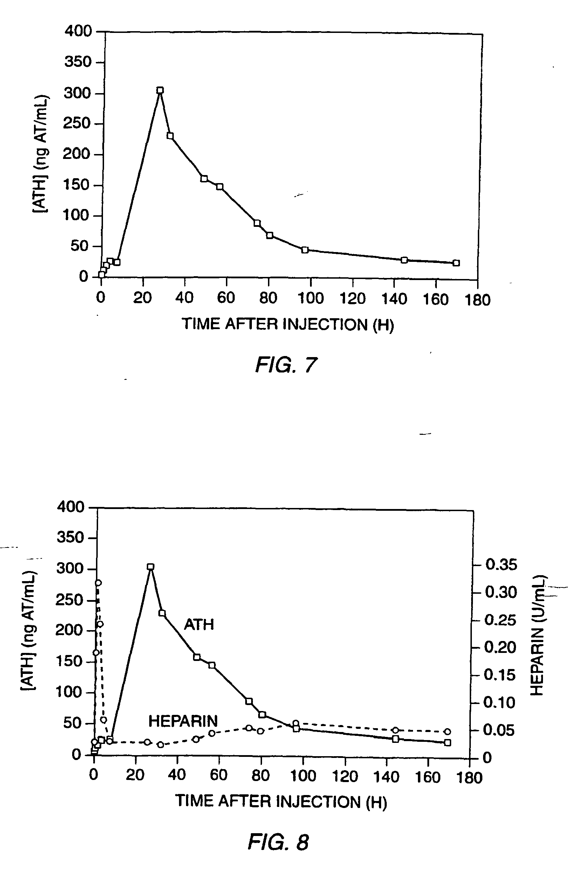

Figure 7 shows the plasma concentrations of the covalent ATH conjugates of the present invention in rabbits after subcutaneous injection.
Figure 8 shows the plasma concentrations of the covalent ATH conjugates of the present invention and heparin in rabbits after subcutaneous injection.
Figure 9 shows AT binding to 100 nM SH or ATH.
Figure 10 shows the activity of ATH and AT+SH in inhibiting thrombin.
Figure 11 shows noncatalytic [□---□] and catalytic [○--○] activities in ATH after chromotography on Sephadex G200.
Figure 12 shows the pharmacokinetics of ATH after intravenous injection, as measured by anti-factor Xa activity.
Figure 13 shows the pharmacokinetic of ATH after intravenous injection as measured by ELISA of Plasma AT.
Figure 14 shows the pharmacokinetic of ATH after subcutaneous injection.
Figure 15 shows the anti-factor Xa activities of BAL after intratracheal instillation of ATH.
Figure 16 shows cumulative blood loss after treatment in a rabbit bleeding ear model.
Figure 17 shows cumulative blood loss after treatment (with outlier removed) in a rabbit bleeding ear model.
Figure 18 shows plasma anti-factor Xa activity in a rabbit bleeding ear model.
Figure 19 shows the change in clot weight for different treatment groups in a rabbit venous thrombosis model.
Figure 20 shows plasma anti-factor Xa activity in a rabbit venous thrombosis model.
Figure 21 shows the clot weights when ATH-grafted, hirudin-grafted, and untreated polyurethane tubing are used in a rabbit perfusion model.
Figure 22 shows shows the clot weights when ATH-grafted, AT-grafted, and untreated polyurethane tubing are used in a rabbit perfusion model.
Figure 23 shows the luminal surface of ATH-treated and untreated tubing after exposure to blood for three hours in rabbits.
Figure 24 shows protein fluorscence scans of AT, AT + SH, and ATH.
Figure 25 shows SH binding to 100 nM AT or ATH.
Figure 26 shows AT binding to 100 nM SH or ATH.

### DESCRIPTION OF THE PREFERRED EMBODIMENT

This invention provides novel covalent conjugates of glycosaminoglycans labelled at their terminal aldose residue with primary amine containing molecules. In particular, this invention provides novel covalent conjugates of heparin (Merck Index, 1980) and fragments thereof with therapeutically significant serine protease inhibitors such as, for example, antithrombin III and heparin cofactor II, therapeutic uses thereof and methods for their preparation. The novel heparin conjugates of this invention are prepared under mild conditions, retain maximal anticoagulant activity compared to intact heparin, and have improved pharmacokinetic properties.

Before describing the invention in greater detail the following definitions are set forth to illustrate and define the meaning and scope of the terms used to describe the invention herein.

The term "hexose" refers to a carbohydrate (C₆H₁₂O₆) with six carbon atoms. Hexoses may be aldohexoses such as, for example, glucose, mannose, galactose, idose, gulose, talose, allose and altrose, whose open chain form contains an aldehyde group. Alternatively, hexoses may be ketoses such as fructose, sorbose, allulose and tagatose, whose open chain form contains a ketone group.

The term "uronic acid" refers to the carboxylic acid formed by oxidation of the primary hydroxyl group of a carbohydrate and are typically named after the carbohydrate from which they are derived. Therefore, oxidation of the C6 hydroxyl of glucose gives glucuronic acid, oxidation of the C6 hydroxyl of galactose gives galacturonic acid and oxidation of the C6 hydroxyl of idose gives iduronic acid.

The term "hexosamine" refers to a hexose derivative in which at least one hydroxy group, typically the C2 hydroxy group, has been replaced by an amine. The amine may be optionally alkylated, acylated (such as with muramic acid), typically by an acetyl group, sulfonated, (O or N-sulfated), sulfonylated, phosphorylated, phosphonylated and the like. Representative examples of hexosamines include glucosamine, galactosamine, tagatosamine, fructosamine, their modified analogs and the like.

The term "glycosaminoglycan" refers to linear chains of largely repeating disaccharide units containing a hexosamine and a uronic acid. The precise identity of the hexosamine and uronic acid may vary widely and representative examples of each are provided in the definitions above. The disaccharide may be optionally modified by alkylation, acylation, sulfonation (O- or N-sulfated), sulfonylation, phosphorylation, phosphonylation and the like. The degree of such modification can vary and may be on a hydroxy group or an amino group. Most usually the C6 hydroxyl and the C2 amine are sulfated. The length of the chain may vary and the glycosaminoglycan may have a molecular weight of greater than 200,000 daltons, typically up to 100,000 daltons, and more typically less than 50,000 daltons. Glycosaminoglycans are typically found as mucopolysaccharides. Representative examples include, heparin, dermatan sulfate, heparan sulfate, chondroitin-6-sulfate, chondroitin-4-sulfate, keratan sulfate, chondroitin, hyaluronic acid, polymers containing N-acetyl monosaccharides (such as N-acetyl neuraminic acid, N-acetyl glucosamine, N-acetyl galactosamine, and N-acetyl muramic acid) and the like and gums such as gum arabic, gum Tragacanth and the like. See Heinegard, D. and Sommarin Y. (1987) *Methods in Enzymology* 144:319-373.

The term "directly covalently linked" refers to a covalent linkage between two species accomplished without the use of intermediate spacer or linkage units. Thus, when a first molecule is referred to as being directly covalently linked to a terminal aldose residue of a glycosaminoglycan via an amino group on the first molecule, this means that the nitrogen atom of the first molecule is bonded directly to an atom of the terminal aldose residue. This bond will be a covalent bond and may be a single, double or triple bond. Therefore, one of skill in the art will understand that heparin conjugates linked to another molecule via initial attachment of spacer groups such as polymethylene diamino linkers to the heparin molecule are not contemplated by this invention.

The term "protein" includes, but is not limited to, albumins, globulins (*e.g.*, immunoglobulins), histones, lectins, protamines, prolamines, glutelins, phospholipases, antibiotic proteins and scleroproteins, as well as conjugated proteins such as phosphoproteins, chromoproteins, lipoproteins, glycoproteins, nucleoproteins.

The term "serpin" refers to a serine protease inhibitor and is exemplified by species such as antithrombin III and heparin cofactor II.

The term "amine" refers to both primary amines, RNH₂, and secondary amines RNH(R').

The term "amino" refers to the group > NH or -NH₂.

The term "imine" refers to the group > C =N- and salts thereof.

As used herein, the terms "treatment" or "treating" of a condition and/or a disease in a mammal, means:
(i) preventing the condition or disease, that is, avoiding any clinical symptoms of the disease;
(ii) inhibiting the condition or disease, that is, arresting the development or progression of clinical symptoms; and/or
(iii) relieving the condition or disease, that is, causing the regression of clinical symptoms.

As used herein, the term "substantially pure" means, an object species is the predominant species present (*i.e.*, on a molar basis it is more abundant than any other individual species in the composition), and preferably a substantially purified fraction is a composition wherein the object species comprises at least about 50 percent (on a molar basis) of all macromolecular species present. Generally, a substantially pure composition will comprise more than about 80 to 90 percent of all macromolecular species present in the composition. Most preferably, the object species is purified to essential homogeneity (contaminant species cannot be detected in the composition by conventional detection methods) wherein the composition consists essentially of a single macromolecular species.

The conditions and diseases treated in the present invention include myocardial infarction and a large array of thrombotic states. These include fibrin deposition found in neonatal respiratory distress syndrome, adult respiratory distress syndrome, primary carcinoma of the lung, non-Hodgkins lymphoma, fibrosing alveolitis, and lung transplants. Also, the present invention can treat either acquired ATIII deficient states such as neonatal respiratory distress syndrome, L-asparaginase induced deficiency, cardiopulmonary bypass induced deficiency and sepsis or congenital ATIII deficient states. In the case of congenital ATIII deficiency, although it is unclear from the literature if any homozygous deficient infant has ever survived to the point of birth, life threatening thrombotic complications with ATIII levels of less than 0.25 Units/ml in heterozygotes requiring ATIII plus heparin may occur in up to 1 or 2 infants per year in the U.S.A.

Other uses of the invention include covalent coating of GAGs on amine containing surfaces such as central venous lines, cardiac catheterization, cardiopulmonary bypass circuits, dialysis circuits, or other external blood contacting instruments, as well as mechanical valves, stents or any *in vivo* prosthesis.

The novel compounds of this invention are prepared by a simple one step process, which provides for direct covalent attachment of the amine of an amine containing moiety (such as, but not limited to, amine containing oligo(poly)saccharides, amine containing lipids, proteins, nucleic acids and any amine containing xenobiotics) to a terminal aldose residue of a glycosaminoglycan. Preferably, the amine containing moiety is a protein possessing a desirable biological activity. The mild non-destructive methods provided herein allow for maximal retention of biological activity of the protein and allow direct linkage of the protein without the need for intermediate spacer groups as follows:

The glycosaminoglycan to be conjugated is incubated with the amine-containing species at a pH suitable for imine formation between the amine and the terminal aldose or ketose residue of the glycosaminoglycan. Terminal aldose and ketose residues generally exist as an equilibrium between the ring closed cyclic hemiacetal or hemiketal form and the corresponding ring opened aldehyde or ketone equivalents. Generally, amines are capable of reacting with the ring opened form to produce an imine (Schiff base). Typically, the aldoses are more reactive because the corresponding aldehydes of the ring open form are more reactive towards amines. Therefore, covalent conjugate formation between amines and terminal aldose residues of glycosaminoglycans provides a preferred method of attaching a species containing an amine to a glycosominoglycan.

The reaction is typically carried out at a pH of about 4.5 to about 9, preferably at about 5 to about 8 and more preferably about 7 to about 8. The reaction is generally done in aqueous media. However; organic media, especially polar hydrophilic organic solvents such as alcohols, ethers and formamides and the like may be employed in proportions of up to about 40% to increase solubility of the reactants, if necessary. Non-nucleophilic buffers such as phosphate, acetate, bicarbonate and the like may also be employed.

The imine is incubated for a sufficient period of time, typically about 1 day to 1 month, more typically about 3 days to 2 weeks, to allow Amadori rearrangement of the intermediate imine. The terminal aldose residues of the glycosaminoglycans conjugated by the methods provided by this invention frequently possess C2 hydroxy groups on the terminal aldose residue, *i.e.,* a 2-hydroxy carbonyl moiety which is converted to a 2-hydroxy imine by condensation with the amine of the species being conjugated to the glycosaminoglycan. In the Amadori rearrangement, which is particularly common in carbohydrates, the α-hydroxy imine (imine at C1, hydroxy at C2) formed by the initial condensation may rearrange to form an α-keto amine by enolization and re-protonation (keto at C2, amine at C1). The resulting α-carbonyl amine is thermodynamically favored over the precursor α-hydroxy imine, thus providing a stable adduct with minimal disruption of the glycosaminoglycan chain. Thus in this embodiment, the invention provides a glycosaminoglycan covalently conjugated at the C1 of the terminal aldose residue of the glycosaminoglycan to an amine containing species via an amine linkage. If desired, the resulting conjugate may be reduced or labelled by reduction of the C2 carbonyl group with a labelling reagent such a radiolabel (*e.g.*, NaB³H₄), see, M.W.C. Hatton, L.R. Berry et al. (1980) *Analytical Biochemistry* 106:417-426. or conjugated to a second amine containing species, such as a fluorescent label.

A variety of different proteins (antibodies, enzymes, receptors, growth factors and the like) may be conjugated to the glycosaminoglycans by the methods disclosed herein. Therefore, this invention provides covalent conjugates of glycosaminoglycans and a variety of proteins. Generally, when proteins are being conjugated to glycosaminoglycans, linkage will occur through the ε-amino groups of lysine residues. Alternatively, linkage may also be accomplished via the N-terminal amine by using a pH at which the ε-amino groups are protonated. In addition, many methods are known to one of skill in the art to introduce an amine functionality into a macromolecule, see, *e.g.,* "Chemistry of Protein Conjugation and Crosslinking", by S. Wong (CRC Press, 1991) and The Organic Chemistry of Biological Compounds", by Robert Barker (Prentice-Hall, 1971).

In particular, the present invention can be applied to a variety of other therapeutically useful proteins where longer half-life and blood coagulation considerations are important. These include blood enzymes, antibodies, hormones and the like as well as related plasminogen activators such as streptokinase and derivatives thereof. In particular, this invention provides conjugates of heparin or dermatan sulfate with antithrombin, heparin cofactor II or analogs of heparin cofactor II, described in U.S. Patent No. 5,118,793.

The methods of the present invention provide glycosaminoglycan conjugates with maximal retention of biological activity. In particular, conjugates of heparin or dermatan sulfate with either ATIII or HCII are provided which possess >60%, typically > 90, more typically > 95%, and most typically ≥ 98% of intact unconjugated heparin antithrombin activity. These conjugates have a bimolecular rate constant for thrombin inhibition of 5 to 100 fold higher, generally 8 to 20 fold higher, and typically almost 10 fold higher than the covalent conjugates reported by Collen.

The method of the present invention provides intact heparin molecules conjugated to antithrombin III or heparin cofactor II. Thus, loss of biological activity associated with fragmentation or other modification of heparin prior to conjugation is avoided. It will be apparent to one of skill in the art that the heparin conjugates of this invention retain their anticoagulant activity because of their preparation from intact heparin.

As described above, the present invention takes advantage of the fact that native (isolated from intestinal mucosa) heparin, as well as dermatan sulfate, already contains molecules with aldose termini which would exist as an equilibrium between hemiacetal and aldehyde forms, a fact apparently unrecognized and unexploited in the art. Thus, we have conjugated heparin or dermatan sulfate to antithrombin serpins by reduction of the single, Schiff base formed spontaneously between the aldose terminus aldehyde on heparin or dermatan sulfate and a lysyl amino on the serpin. The heparin or dermatan sulfate is unmodified (unreduced in activities) prior to conjugation and is linked at one specific site at one end of the molecule without any unblocked activation groups or crosslinking of the serpin. Heparin has been covalently linked to ATIII or HCII and dermatan sulfate has been covalently linked to HCII. Conjugation of other GAGs (such as heparan sulfate) to serpins or other proteins (such as albumin) is possible by this method. For example, dermatan sulfate has been conjugated to albumin using the methods disclosed herein.

In another aspect of this invention we have also produced covalent complexes by simply mixing heparin and ATIII in buffer and allowing a keto-amine to spontaneously form by an Amadori rearrangement between the heparin aldose terminus and an ATIII lysyl amino group. Thus, this invention provides methods of using the Amadori rearrangement to prepare conjugates of glycosaminoglycans to proteins. This is a particularly mild and simple method of conjugation, hitherto unrecognized in the art for conjugating such molecules, which minimizes the modification of the glycosaminoglycan, thus maximizing the retention of its biological activity.

Another aspect of this invention provides covalent conjugates of glycosaminoglycans, particularly of heparin, end-labelled with an amine containing protein at the terminal aldose residue of the glycosaminoglycan. For example, heparin and ATIII are linked directly together so that the active pentasaccharide sequence for ATIII on the heparin is in close proximity for binding. This is one of the fundamental reasons for making a covalent heparin-ATIII complex, as heparin accelerates inhibition through ATIII only if ATIII can bind the active sequence. It is notable that ATH has the unique property that the H in the conjugate stoichiometrically activates the endogenous AT while catalytically activating exogenous AT. Typically, one amine containing protein will be attached to each glycosaminoglycan. However, it will be apparent that the ratio of amine containing species to glycosaminoglycan may be reduced below one by adjusting the molar ratios of the reactants or the time of the reaction.

Glycosaminoglycans are available in a variety of forms and molecular weights. For example, heparin is a mucopolysaccharide, isolated from pig intestine or bovine lung and is heterogenous with respect to molecular size and chemical structure. It consists primarily of (1-4) linked 2-amino-2-deoxy-α-D-glucopyroanosyl, and α-L-idopyranosyluronic acid residues with a relatively small amount of β-D-glucopyranosyluronic acid residues. It contains material with a molecular weight ranging from about 6,000 to about 30,000. The hydroxyl and amine groups are derivatized to varying degrees by sulfation and acetylation.

Heparin molecules can also be classified on the basis of their pentasaccharide content. About one third of heparin contains chains with one copy of the unique pentasaccharide (*see,* Choay, *Seminars in Thrombosis and Hemostasis* 11:81-85 (1985) which is incorporated herein by reference) with high affinity for AT, whereas a much smaller proportion (estimated at about 1 % of total heparin) consists of chains which contain more than one copy of the high affinity pentasaccharide (*see,* Rosenberg *et al., Biochem. Biophys. Res. Comm.* 86:1319-1324 (1979) which is incorporated herein by reference). The remainder (approx. 66%) of the heparin does not contain the pentasaccharide. Thus, so called "standard heparin" constitutes a mixture of the three species, "high affinity" heparin is enriched for species containing at least one copy of the pentasaccharide, and "very high affinity" heparin refers to the approximately 1 % of molecules that contain more than one copy of the pentasaccharide. These three species can be separated from each other using routine chromatographic methods, such as chromatography over an anti-thrombin affinity column (*e*.*g*., Sepharose-AT; *see, e.g.,* Lam *et al*., *Biochem. Biophys*. *Res. Comm.* 69:570-577 (1976) and Homer *Biochem. J.* 262:953-958 (1989)).

One advantage of forming a conjugate between heparin and a species containing at least one primary amino group (*e.g.*, ATIII) using the slow glycation process disclosed herein, is the apparent selection for heparin chains having two pentasaccharides. Thus, for example, ATH prepared by the method of the invention appears to be enriched for heparin species containing two pentasaccharides. When standard heparin (containing approximately 1 % of two-pentasaccharide heparin) is used as a starting material, usually more than 10% of the resulting ATH comprises two-pentasaccharide heparin, more often more than about 20%, frequently more than 35%, and often more than about 50% of the ATH comprises two-pentasaccharide heparin.

Without intending to be bound by any particular mechanism, one explanation for the apparent selection of very high affinity heparin is because the incubation mixture contains a 200-fold molar excess of heparin. During the incubation process, only heparin chains containing high affinity pentasaccharides close to a terminal aldose bind to the AT for a sufficiently long period of time to allow covalent attachment to occur. Therefore there is a selective interaction between AT and the very high affinity heparin chains.

This enrichment may account for several useful properties of ATH. The ATH of the invention activates the AT to which it is conjugated, in a stoichiometric fashion, but activates exogenous AT in a catalytic fashion. Thus, the heparin within the ATH complex acts catalytically both when ATH is administered as systemic anticoagulant and when ATH is used to coat surfaces to render them non-thrombogenic. The method of the invention produces an ATH complex with very high specific anti-factor IIa activity. In addition, the second pentasaccharide chain in the ATH complex can react with exogenous AT molecules, thereby allowing the conjugated heparin to have catalytic activity. Moreover, the heparin in the ATH complex can be orientated in such a way that the pentasaccharide is available to bind and activate circulating AT molecules when the ATH complex is bound to the prosthetic surface.

It will be appreciated that a heparin conjugate of interest (*e.g.*, ATH) can also be produced by incubating a species containing at least one primary amino group (*e.g.,* ATIII) with purified very high affinity heparin (*i.e.*, containing two pentasaccharide groups) or a fraction enriched for very high affinity heparin.

Though this invention has been illustrated primarily with respect to heparin, it is apparent that all glycosaminoglycans, irrespective of their molecular weight and derivatization, may be conjugated by the methods disclosed herein, provided they possess a terminal aldose residue. Conjugates of all such glycosaminoglycans and their preparation by the methods disclosed herein are within the scope of this invention. For example, conjugates of heparin derivatized with phosphates, sulfonates and the like as well as glycosaminoglycans with molecular weights less than 6,000 or greater than 30,000 are within the scope of this invention.

In clinical practice, the novel heparin conjugates of the present invention may be used generally in the same manner and in the same form of pharmaceutical preparation as commercially available heparin for clinical use. Thus, the novel heparin conjugates provided by the present invention may be incorporated into aqueous solutions for injection (intravenous, subcutaneous and the like) or intravenous infusion or into ointment preparations for administration via the skin and mucous membranes. One skilled in the art will recognize that all forms of therapy, both prophylactic and curative, either currently known or available in the future, for which heparin therapy is indicated may be practiced with the novel heparin conjugates provided by this invention.

The heparin conjugates of this invention find particular utility in the treatment of neonatal and adult respiratory distress syndrome (RDS). In contrast to the use of noncovalent heparin-ATIII complexes, the use of the covalent heparin conjugates of the present invention prevents loss of heparin in the lung space by dissociation from ATIII. In this case, a solution of covalent complex in a physiologic buffer could be delivered as an atomized spray down the airway into the lung via a catheter or puffer. Due to its large size, ATH will remain in the alveoli for a longer period of time. ATH is also useful for treatment of idiopathic pulmonary fibrosis (more than two days).

Long term use in the circulation could be carried out by either intravenous or subcutaneous, preferably intravenous, injection of the complex in a physiologic buffer. The covalent conjugates of this invention may also be used in the treatment of acquired ATIII deficient states characterized by thrombotic complications such as cardiopulmonary bypass, extracorporeal molecular oxygenation, etc. because a longer 1/2 life of the covalent complex would mean fewer treatments and less monitoring. Additionally, this invention provides conjugates for prophylactic treatment of adult patients at risk for deep vein thrombosis.

The ATH conjugate of this invention has numerous advantages over uncomplexed AT and SH. Since AT is covalently linked to SH, non-specific binding of ATH to plasma proteins will be less than SH, resulting in less inter-individual variation in dose response to ATH than there is to SH. The longer half-life of ATH after intravenous injection in humans means that a sustained anticoagulant effect may be obtained by administering ATH less frequently than is required for uncomplexed AT and SH. ATH is a much more effective inactivator of thrombin and factor Xa than AT, and is expected to be effective when used in much lower concentrations than AT in patients with AT deficiency. In addition, ATH can access and inhibit thrombin bound to fibrin. Finally, when linked (*e*.*g*., covalently linked) to prosthetic surfaces (*e*.*g*., endovascular grafts), ATH has shown much greater antithrombotic activity *in vivo* than covalently linked AT or covalently linked hirudin.

Premature infants have a high incidence of respiratory distress syndrome (RDS), a severe lung disease requiring treatment with assisted ventilation. Long term assisted ventilation leads to the onset of bronchopulmonary dysplasia (BPD) as a result of lung injury which allows plasma coagulation proteins to move into the alveolar spaces of the lung. This results in the generation of thrombin and subsequently fibrin. The widespread presence of fibrin within the lung tissue and airspaces is consistently observed in infants dying of RDS. This fibrin gel within the airspace impairs fluid transport out of the lung airspaces resulting in persistent and worsening pulmonary edema. This invention provides conjugates useful in novel therapies for the treatment of such fibrin-mediated diseases in lung tissue by preventing intra-alveolar fibrin formation by maintaining an "anti-thrombotic environment" and/or enhancing fibrinolysis within lung tissue, thereby decreasing the fibrin load to the air spaces of the lung.

The heparin conjugates will be delivered directly to the airspaces of the lung via the airway prophylactically (before the baby takes its first breath). This ensures that the antithrombotic agent is available directly at the site of potential fibrin deposition and that the bleeding risk associated with systemic antithrombotic therapies is avoided. In addition, the antithrombotic agent will already be present in the lung prior to the start of the ventilatory support which is associated with the initial injury, *i.e.*, unlike systemic antithrombin administration where crossing of the administered drug to the lung airspace does not occur until after lung injury. Since heparin is covalently attached to ATIII it will remain in the lung airspaces. It can also be an adjunctive therapy to the surfactants currently administered to prevent RDS and BPD. By "lung surfactant" is meant the soap-like substance normally present in the lung's airspaces whose main role is to prevent collapse of the airspace. The conjugates can also be delivered repeatedly via the endotracheal tube or as an inhaled aerosol. Adjunctive therapy can also be practiced with asthma medications by inhaler (*e.g.*, anti-inflammatory steroids such as beclomethasone dipropionate), other anti-asthmatics such as cromolyn sodium (disodium salt of 1,3-bis(2-carboxychromon-5-yloxy)-2-hydroxypropane, INTAL^{®}) and bronchodilators such as albuterol sulfate.

A variety of other diseases associated with elevated thrombin activity and/or fibrin deposition can be treated by administration of the conjugates of this invention. The inflammatory processes involved in adult respiratory distress syndrome are fundamentally similar to neonatal RDS and can be treated by the antithrombotic therapy described. Spontaneous lung fibrosis has also been shown to have activation of the coagulation/fibrinolytic cascades in the lung airspaces. Fibrotic disease of the lung is often a side effect associated with cancer chemotherapy and the RDS antithrombotic administration of the covalent heparin conjugates of this invention can be administered prophylactically prior to cancer chemotherapy to prevent lung fibrosis. Administration is repeated after chemotherapy in order to ensure no fibrin formation. A decrease in antithrombin III activity and an increase in thrombin activity in sepsis is also well documented. Sepsis is the most common risk factor for developing adult RDS. Thus, the heparin conjugates of this invention can be used to reduce the mortality associated with septic shock.

The conjugates of this invention are administered at a therapeutically effective dosage, *i.e*., that amount which, when administered to a mammal in need thereof, is sufficient to effect treatment, as described above (for example, to reduce or otherwise treat thrombosis in the mammal, or to inactivate clot-bound thrombin, or to inhibit thrombus accretion). Administration of the active compounds and salts described herein can be via any of the accepted modes of administration for agents that serve similar utilities.

The level of the drug in a formulation can vary within the full range employed by those skilled in the art, *e.g.*, from about 0.01 percent weight (%w) to about 99.99 % w of the drug based on the total formulation and about .01% w to 99.99 % w excipient. Preferably the drug is present at a level of about 10%w to about 70%w.

Generally, an acceptable daily dose is of about 0.001 to 50 mg per kilogram body weight of the recipient per day, preferably about 0.05 to 25 mg per kilogram body weight per day, and most preferably about 0.01 to 10 mg per kilogram body weight per day. Thus, for administration to a 70 kg person, the dosage range would be about 0.07 mg to 3.5 g per day, preferably about 3.5 mg to 1.75 g per day, and most preferably about 0.7 mg to 0.7 g per day depending upon the individuals and disease state being treated. Such use optimization is well within the ambit of those of ordinary skill in the art. In the case of ATH, the long half-life allows the compound to be administered less frequently than SH (*e.g.*, once or twice weekly).

Administration can be via any accepted systemic or local route, for example, via parenteral, intravenous, nasal, bronchial inhalation (*i.e.*, aerosol formulation), transdermal or topical routes, in the form of solid, semi-solid or liquid dosage forms, such as for example, tablets, suppositories, pills, capsules, powders, solutions, suspensions, aerosols, emulsions or the like, preferably in unit dosage forms suitable for simple administration of precise dosages. Administration by intravenous or subcutaneous infusion is usually preferred. Most usually, aqueous formulations will be used. The conjugate is formulated in a non-toxic, inert, pharmaceutically acceptable carrier medium, preferably at a pH of about 3-8, more preferably at a pH of about 6-8. Generally, the aqueous formulation will be compatible with the culture or perfusion medium. The compositions will include a conventional pharmaceutical carrier or excipient and a conjugate of the glycosaminoglycan, and in addition, may include other medicinal agents, pharmaceutical agents, carriers, adjuvants, etc. Carriers can be selected from the various oils, including those of petroleum, animal, vegetable or synthetic origin, for example, peanut oil, soybean oil, mineral oil, sesame oil, and the like. Water, saline, aqueous dextrose or mannitol, and glycols are preferred liquid carriers, particularly for injectable solutions. Suitable pharmaceutical carriers include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the like. Other suitable pharmaceutical carriers and their formulations are described in Remington's Pharmaceutical Sciences by E. W. Martin (1985).

If desired, the pharmaceutical composition to be administered may also contain minor amounts of non-toxic auxiliary substances such as wetting or emulsifying agents, pH buffering agents and the like, such as for example, sodium acetate, sorbitan monolaurate, triethanolamine oleate, etc.

The compounds of this invention are generally administered as a pharmaceutical composition which comprises a pharmaceutical excipient in combination with a conjugate of the glycosaminoglycan. The level of the conjugate in a formulation can vary within the full range employed by those skilled in the art, *e.g.*, from about .01 percent weight (%w) to about 99.99%w of the drug based on the total formulation and about .01 %w to 99.99%w excipient. Preferably, the formulation will be about 3.5 to 60% by weight of the pharmaceutically active compound, with the rest being suitable pharmaceutical excipients.

The compounds of the invention, particularly ATH, can be used to reduce the thrombogenicity of internal and extracorporal devices that contact blood, and find special use for coating thrombogenic prosthetic surfaces and medical devices. As used herein, "prosthetic devices" and "medical devices" refers to any natural or synthetic material that is implanted into a patient or otherwise comes into contact with blood and for which it would be desirable to reduce blood coagulation. Thus, these terms encompass endovascular tubing, arterial and central venous lines, cardiac catheters, cardiopulmonary bypass circuits, dialysis circuits, or other external blood contacting instruments, as well as pacemaker leads, arterial and venous catheters for cannulation of large vessels thrombectomy catheters, sutures, blood filters, intravenous lines, mechanical valves, stents, artificial kidneys, lungs, hearts, and livers or any *in vivo* prosthesis, especially those made from a natural or synthetic polymer or polymers.

Materials used in prosthetic devices include Ioplex materials and other hydrogels such as those based on 2-hydroxyethyl methacrylate or acrylamide, and poly ether polyurethane ureas (PEUU) including Biomer (Ethicon Corp.) and Avcothane (Avco-Everrett Laboratories). The materials used most frequently for tubular applications are polyethylene, polypropylene, polytetrafluoroethylene (Gore-Tex), poly(vinylchloride), polydimethylsiloxane, an ethylene-acrylic acid copolymer, knitted or woven Dacron, polyester-polyurethane, polyurethane, polycarbonate-polyurethane (Coretbane^{™}), polyamide (Nylon) and polystyrene. Additional compounds used in prosthetics and biomedical devices which come into blood contact are described in Kirk-Othmer Encyclopedia of Chemical Technology, 3rd Edition 1982 (Vol. 19, pp. 275-313, and Vol. 18, pp. 219-2220) and van der Giessen *et al., Circulation* 94:1690-1997 (1996).

In general, the composition of the invention, *e.g.*, ATH, will be covalently attached to the polymer of the device. Methods for covalent attachment are well known and will vary depending on the nature of the polymeric material. In general, see Hermanson, Mallia and Smith, *Immobilized Affinity Ligand Techniques,* Academic Press (1992). It will be appreciated that other polymers and materials, possibly including some not yet discovered, will be suitable for linkage to ATH or other conjugates of the invention.

In a preferred embodiment, a polyurethane-polycarbonate material is coated with ATH. This coating is carried out in three steps. First, the polymer is activated. Activation can be accomplished by treatment with an oxidant (*e.g.*, sodium hypochlorite, NaOCl) or a reductant (*e.g.*, Lithium Aluminum Hydride). Second, a monomer (allyl glycidyl ether) is grafted onto the surface by reacting the activated tubing with an initiator (Na₂S₂O₄) and a monomer (*e.g.*, allyl glycidyl ether, acrolein, or another monomer with a functional group joined to an alkene) that can further react with the compounds of the invention, *e.g.*, ATH. Third, the compound to be linked, (*e.g.*, ATH or other anticoagulants that have groups, such as, an amino group, that can react with the functional group of the monomer) is linked to the monomer. One advantage of this method is that it does not involve any manipulation of ATH and does not alter its anticoagulant activity.

The conjugates of the invention are also useful as molecular weight standards for analysis of unknown samples.

The following examples are given to enable those of skill in the art to more clearly understand and practice the invention.

### Materials

In the following methodology, unless otherwise noted, "Standard heparin" refers to heparin from commercial sources. High affinity heparin is a heparin fraction in which all of the molecules bind to ATIII.

Heparin was from porcine intestinal mucosa (Sigma Chem Co U.S.A.). Dermatan sulfate was from porcine intestinal mucosa (Mediolanum farmaceutici S.p.A., Italy). ATIII was from human plasma (Bayer Inc.) HCII was from human plasma (Affinity Biologicals).

### EXAMPLE I

### Preparation of Covalent Conjugates between GAGs and Serpins

Reactions to form covalent complexes between the glycosaminoglycan (GAG) and serpin, for example ATIII or HCII, involved incubation of GAG (5mg-70mg) with the serpin (0.5mg-3mg) in 1mL of sterile filtered buffer (0.3M phosphate 1M NaCl, pH 8.0 or 0.02M phosphate 0.15M NaCl, pH 7.3) containing 0.05M sodium cyanoborohydride at 35°C to 45°C, preferably 40°C, in a sealed plastic tube (polycarbonate, polypropylene, etc). Omitting the sodium cyanoborohydride allowed formation of covalent complexes via Amadori rearrangement which could be radiolabeled by later addition of tritium labelled sodium borohydride. Incubation times ranged from 3 days to 2 weeks. Purification of the covalent product was achieved by a variety of methods. Purification procedures are described in U.S. Patent No. 5,308,617, U.S. Patent No. 4,623,718 and *FEBS Letters* 143(1):96-100, 1982, all incorporated by reference. Gel filtration on Sephadex G-200 using 2M NaCl produced a high molar mass fraction containing covalent complex that was essentially void of free serpin. This fraction was further purified by electrophoresis on a 7.5 % polyacrylamide gel at pH 8.8 using nondenaturing conditions (no sodium dodecyl sulfate), cutting out the section of gel containing only complex and elution of the product from the cut up section of gel by incubation in buffer (3.0g/L tris (hydroxymethyl) aminomethane 14.4g/L glycine pH8.8) at 23 degrees C.

Alternatively, the antithrombin-heparin conjugate (ATH) was also purified in one step from the reaction mixture by hydrophobic chromatography on butyl-agarose (Sigma Chemical Company, Milwaukee, WI). In 2.5M ammonium sulfate, ATH and ATIII bound to butyl-agarose beads while heparin did not. Adjusting the ammonium sulfate concentration from 2.5M to 1.8M allowed pure ATH to be eluted from the beads while ATIII remained bound.

Also, ATH and ATIII bound to butyl-agarose could be eluted together by adjusting the ammonium sulfate concentration to less than 1.5M followed by separation of the ATH from ATIII on DEAE Sepharose Fast Flow beads (Pharmacia Biotech, Uppsala Sweden). ATH and ATIII eluted from butyl-agarose were dialyzed versus 0.01M Tris-HCl pH 8.0 buffer prior to binding to the DEAE beads and the bound ATIII eluted with 0.2M NaCl in buffer while ATH was eluted by NaCl concentrations of 0.4M to 2.0M. In this way, ATH of different molecular weights and charges could be isolated, depending on the NaCl concentration used. Concentration of the purified ATH was done at 4°C by dialysis in tubing, with a 12000-14000 molar mass cut off, under nitrogen pressure (1 atmosphere).

ATH produced in 0.02M phosphate, 0.15M NaCl, 0.05M sodium cyanoborohydride pH 7.3 and purified using elution of the complex from a cut out section of gel following nondenaturing electrophoresis, yielded material in which the molar ratio of ATIII:H in the complex was 1:1.1 and > 99 % was active.

### EXAMPLE III

### Characterization of GAG-Serpin Conjugates

### 1. Biological Activity

Anti-Xa activity measured by Collen et al. and Bjork et al. for their respective ATH preparations was carried out by (pre)incubation of the ATH with Xa followed by determination of residual activity of Xa with S-2222 (N-benzoyl-isoleucyl-glutamyl-glycyl-arginyl-paranitroanilide (from Chromogenix, Sweden)). The percent of ATH molecules with activity (as determined by amount of Xa inhibited) is reported in Table I. Anti-IIa activity was measured for our ATH by titration with different amounts of IIa (thrombin). The amount of IIa inhibited by a given mass concentration of ATH (mass determined by analysis using unmodified starting heparin) was determined by measuring residual activity against S-2238 (D-phenylalanyl-pipecolyl-arginyl-paranitroanilide (from Chromogenix, Sweden)).

### Inhibition of Thrombin Activity

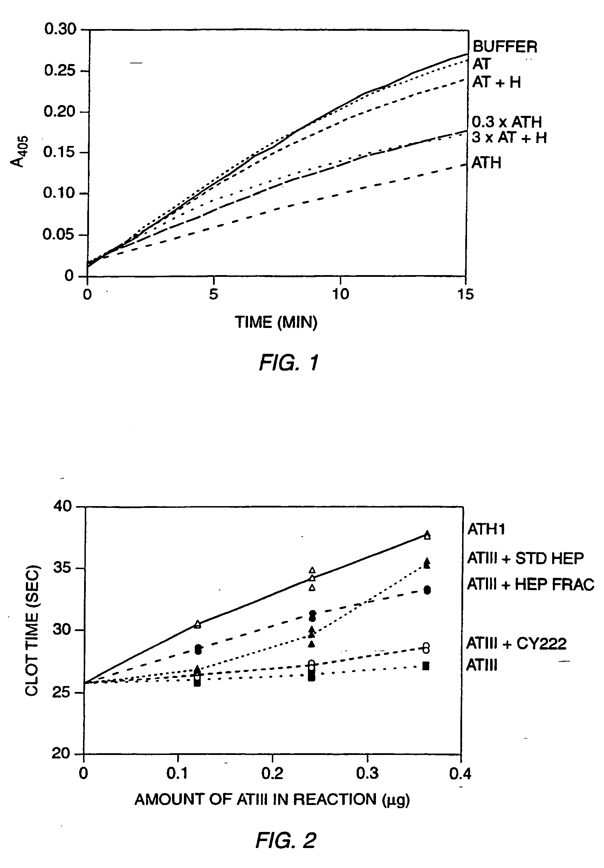

The inhibition of the reaction of bovine thrombin with the chromogenic substrate S-2238 was studied. All operations were carried out at 23°C. Thrombin was added, with mixing, to a solution containing the material to be tested and S-2238 dissolved in 0.036M sodium acetate 0.036M sodium barbital, 0.145M NaCl pH 7.4 buffer in an eppendorf tube (the final thrombin concentration was 0.045 I.U./ml and the final S-2238 concentration was 28.3 µg/ml). The resultant solution was transferred to a quartz cuvette and absorbance readings at 405 nm taken over time (zero time being 30 sec after addition of the thrombin). The reaction concentration of the ATIII in either the ATH, ATIII or ATIII+H (heparin) reactions was 8.8 nM. The [ATIII] in the 0.3 x ATH and 3 x AT + H reactions was 2.7 nM and 27 nM, respectively. In reactions where heparin was used, it was present in equimolar concentrations to the ATIII in that experiment. The results are shown in Figure 1 and show that the ATH conjugates of the present invention are more effective than free ATIII and heparin.

Thrombin was from Parke-Davis. S-2238 was from Chromogenix (Sweden). Standard Heparin (Leo Laboratories) was used.

### Reaction with Fibrinogen and Thrombin

The ability of bovine thrombin to clot human fibrinogen was inhibited by various ATIII containing mixtures as follows. An ATIII containing sample was mixed with fibrinogen in 0.15M NaCl in a plastic tube at 37°C. After 1 min, thrombin was added (the final fibrinogen concentration was 0.2 mg/ml and the final thrombin concentration was 1 I.U./ml) and a clock was started. The time was recorded for the first appearance of a clot on the end of a nichrome wire loop used for agitation. The results are shown in Figure 2 and show that the ATH conjugates are more effective at preventing clotting. The following abbreviations are used.
- ATH1 =: preparation #1 of ATIII-Heparin conjugate(as described in Example 1)
- STD Hep =: standard heparin (LEO laboratories)
- HEP FRAC =: low molecular weight fraction (≈ 7000MW, produced by gel filtration) of standard heparin
- CY222 =: low molecular weight heparin fragment produced by nitrous acid (average ≈ 2500MW, produced by Choay Laboratories)

Thrombin was from Parke-Davis; Fibrinogen was from Connaught Laboratories. ATIII was purified from human plasma. In ATIII + heparin mixtures, the protein and GAG content were equivalent on a mass basis (only 1 in 3 standard heparin molecules bind ATIII).

### Effect of added Heparin on Rate of Inhibition of Thrombin Activity by Covalent ATIII-Heparin Conjugates (ATH)

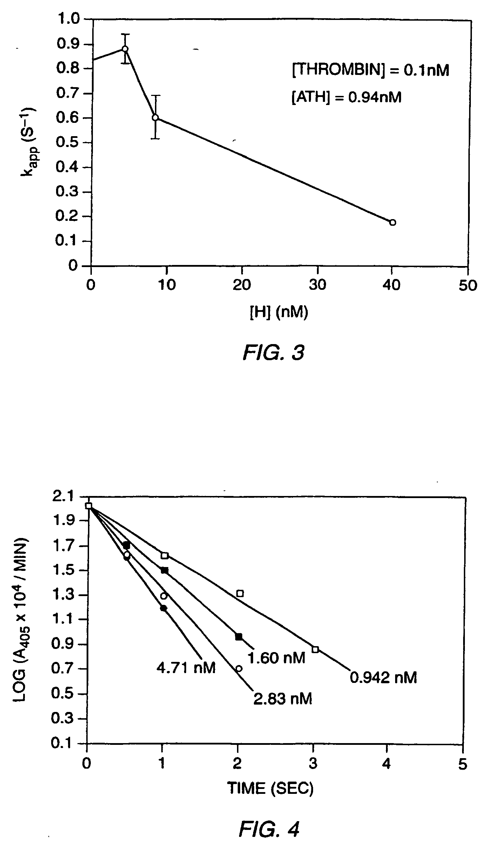

The ability of standard heparin to affect the inhibition of the human thrombin by ATH was tested. The buffer used was 0.1M Tris-HCl, 0.15M NaCl, 1.5 µM bovine albumin pH 7.6. ATH and varying amounts of heparin in buffer were placed in a 8 mm diameter, flat bottomed, polycarbonate, plastic tube equipped with a stirring bar rotating at 500-1000 rpm, all in a 37°C water bath. Human thrombin was added immediately as a clock was started. After a time ranging from 0.5 to 5 sec, thrombin inhibition was stopped by addition of a solution of excess polybrene and S-2238. Residual thrombin activity for S-2238 (A₄₀₅/min) was measured in a quartz cuvette at 37°C. The results are shown in Figure 3. A semi-log plot of residual thrombin activity (Log (A₄₀₅x10⁴/min)) versus time (sec) was constructed for each heparin concentration used. The apparent rate constant (kₐₚₚ (S⁻¹)) was calculated as ln 2 divided by the time at which ½ of the starting thrombin activity was inhibited. The kₐₚₚ for each heparin concentration is plotted.

Bovine albumin was from Sigma Chemical Company, Human thrombin was from Enzyme Research Laboratories (U.S.A.), S-2238 was from Chromogenix (Sweden) and heparin was from Leo Laboratories, Canada. All concentrations quoted on Figure 3 are reaction concentrations just prior to polybrene-S-2388 addition.

### Determination of Rates of Thrombin Inhibition by ATH

The experimental procedure and calculation of the semi-log plot was the same as the experiments described above for Figure 3 except that no exogenous heparin was added and the concentration of ATH was varied as shown. The results are shown in Figure 4.

### Inhibition of Thrombin + ATH reaction by FPR-Thrombin

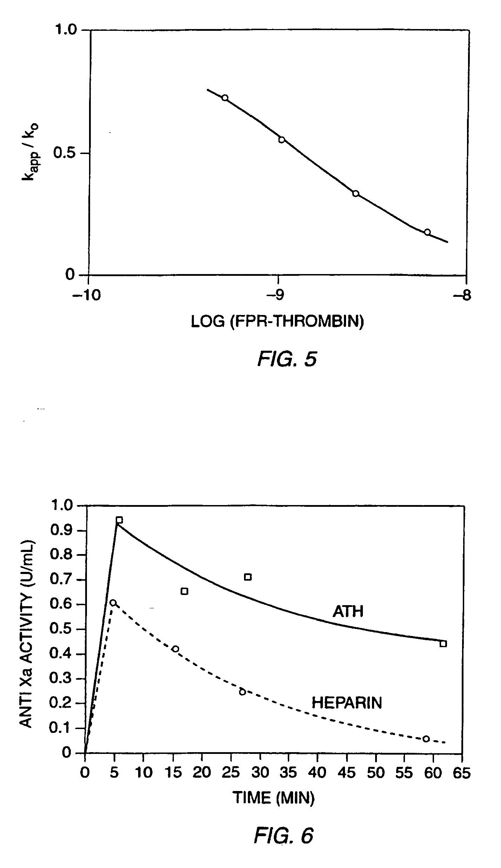

FPR-thrombin is thrombin inhibited by phenylalanyl-prolyl-arginyl peptide covalently bonded to its active serine. FPR-thrombin can competitively inhibit the reaction of thrombin with ATH by binding to the heparin chain although it cannot react with the ATIII portion. The experimental procedure and calculation of kₐₚₚ was the same as for the experiments for Figure 3 except that varying amounts of FPR-thrombin were tested instead of heparin (no exogenous heparin added). The constant kₒ was the kₐₚₚ value with no FPR-Thrombin added. Results are shown in Figure 5.

### Bimolecular and 2nd order rate constants and effect of added Heparin on Rate of Inhibition of Thrombin by ATH

The procedure for the results for added heparin are given as determined from the results used for Figure 3. To determine the rate constants, the method of Hoylaerts et al. in *J. Biol. Chem.* 259(9):5670-5677 (1984) was used. To calculate the bimolecular rate constant, k₂ and Kᵢ were determined as follows. The kₐₚₚ values for each curve for each ATH concentration used were determined for 3 separate experiments, of which Figure 4 is a typical example. For each experiment, a plot of 1/kₐₚₚ versus 1/[ATH] was constructed. The intercept of the 1/kₐₚₚ axis was equal to 1/k₂ and the intercept of the 1/[ATH] axis was equal to 1/Kᵢ. In each case, the bimolecular rate constant was calculated as k₂/Kᵢ and the average of 3 experiments is reported. For the second order rate constant (k₁), k₁(off rate), or IC₅₀ for FPR-thrombin competition ([FPR-Thrombin] at which kₐₚₚ/k₀ = 0.5) was determined for each curve for each of 3 experiments, of which Figure 5 is a typical example. The averages, for the three k₂ and Kᵢ values measured were used to calculate the second order rate constant for each k₋₁ value, given the following formula. Second order rate constant = k₁ = (k₋₁ +k₂)/Kᵢ. The average is reported. Results are shown in Table 2. Error values are expressed as ± 2 times the standard error of the mean.

**TABLE 2**

| PRODUCT | BIMOLECULAR RATE CONSTANT (M⁻¹s⁻¹) | 2nd ORDER RATE CONSTANT (M⁻¹s⁻¹) | EFFECT OF >10 FOLD MOLAR EXCESS ADDITION OF HEPARIN |
|---|---|---|---|
| BERRY et al. | 1.3x10⁹ ± 2x10⁸ | 3.1x10⁹ ± 4x10⁸ | INHIBITION OF ANTI-IIa ACTIVITY |
| COLLEN et al. | 3x10⁴ | 6.7x10⁸ | ANTI-Xa ACTIVITY DOUBLED |
| BJORK et al. | ----- | ----- | |

### Pharmacokinetics of Covalent ATIII-Heparin Conjugates

### 1. Plasma Clearance of ATH and Heparin After Intravenous Injection in Rabbits

Purified ATH and standard heparin (Sigma) were injected into the ear vein of separate rabbits. Equivalent amounts (by mass of heparin) were injected. At various times, blood samples were withdrawn from the ear artery of each rabbit into sodium citrate (9 parts blood to 1 part 3.8% (m/v) trisodium citrate). Each sample was centrifuged at 3000g and the resultant plasma supernatants analyzed for anti-Xa activity using an ACL300 machine (Coulter U.S.A.) for automation. The procedure employed a Stachrom Heparin kit (Diagnostica Stago, France). Briefly, each sample of plasma to be tested was mixed with buffer containing bovine ATIII and incubated with bovine factor Xₐ at 37°C for 30 sec followed by a 30 sec incubation with the chromogenic substrate CBS 31.39 (N-(methylsulfone)-D-leucyl-glycyl-arginyl-paranitroanilide (from Diagnostica Stago, France)), after which reaction was stopped by addition of acetic acid. The absorbance at 405 nm was then measured. A standard curve, generated using standard heparin, was used to determine the anti-Xₐ activity in the plasma samples in terms of I.U./ml of heparin. Results are shown in Figure 6. The ATH half life was observed to be 53 minutes and the free heparin half life was observed to be 17 minutes.

### 2. Pharmacokinetics in plasma after subcutaneous injection in rabbits.

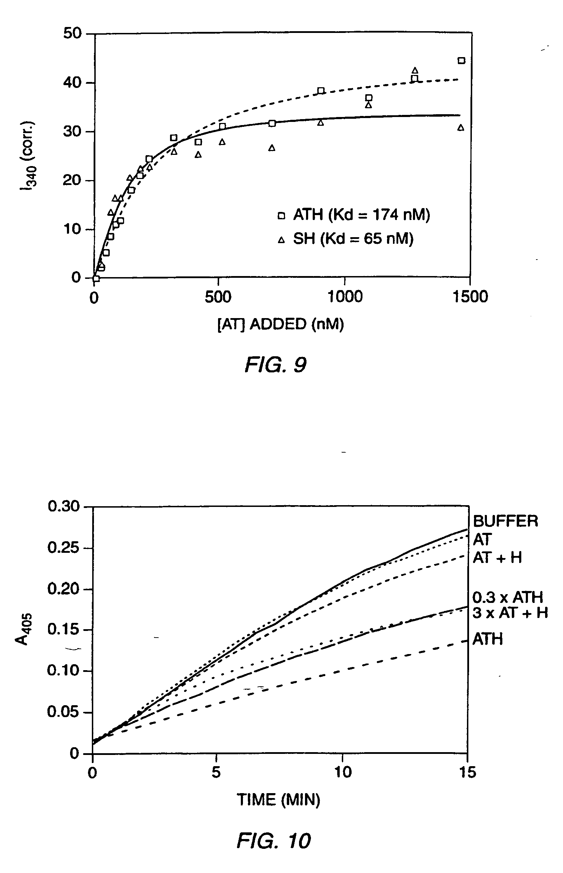

Rabbits were injected under the skin behind the neck and blood sampling for plasma analysis being done at various times as described above for Figure 6. ATH was detected using an ELISA kit for ATIII from Affinity Biologicals (Hamilton, Canada). Briefly, ATH from sample plasmas was captured on plastic wells coated with sheep anti-human ATIII polyclonal antibodies. Peroxidase conjugated affinity purified anti-human ATIII antibodies (polyclonal) were applied to the wells and, after rinsing, color developed with H₂O₂/O-phenylenediamine substrate for 10 min. After terminating substrate reaction with H₂SO₄, the absorbance at 490 nm was measured. Standard curves of ATH or human ATIII in pooled normal rabbit plasma were used to determine the ng of human ATIII/ml. The rabbit's own ATIII did not interfere significantly, as the antibody used was selective for human ATIII. Results are shown in Figure 8. In a separate experiment, when ATIII and heparin (noncovalent conjugate) was injected subcutaneously, ATIII (detected by ELISA) appeared in plasma with the same profile as ATH, but no heparin activity was observed.

### 2. Structural Characterization

### A. General Structural Characteristics

The procedure to determine the molar ratio of Hep:AT in the heparin-antithrombin conjugates (ATH) was by densitometry of SDS gels (standard procedures) stained for either heparin (alcian blue/silver) or ATIII (Coomassie blue) compared with the corresponding standards. The activating groups per GAG molecule is by definition 1 (one aldose terminus per GAG chain).

The molecular weight range was determined from comparison of stained ATH, HCH, HCD with prestained standards on SDS polyacrylamide gels.

Characteristics of antithrombin-heparin conjugates(ATH) and heparin cofactor II-heparin (HCH) and heparin cofactor II-dermatan sulfate (HCD) conjugates are shown below in Table 1.

**TABLE 1**

| CHARACTERISTICS OF COVALENT ATH PRODUCTS | | | | | |
|---|---|---|---|---|---|
| PRODUCT | MOLAR RATIO HEP:AT | ACTIVATING GROUPS PER HEP | MOL. WT. (SDS PAGE) | ANTI-Xa* | ANTI-IIa* |
| BERRY et al. | 1.1 | 1 | 69kD-100kD | | >98% |
| COLLEN et al. | 0.8-0.9 | 2.1 | | 65% | ≤65% |
| BJORK et al. | 0.7 | 1 | | 82% | UNDETECTABLE |
| * ACTIVITY OF HEPARIN IN COMPLEX COMPARED TO UNMODIFIED STARTING HEPARIN | | | | | |

| CHARACTERISTICS OF COVALENT HCH AND HCD PRODUCTS | | | | | |
|---|---|---|---|---|---|
| PRODUCT | MOLAR RATIO GAG:AT | | ACTIVATING GROUPS PER GAG | MOL. WT. (SDS PAGE) | ANTI IIa* |
| HCH | 1.1 | | 1 | 70kD-115kD | >90% |
| HCD | 1.4 | | 1 | 78kD-150kD | >90% |
| * PERCENT OF MOLECULES ACTIVE AGAINST IIa | | | | | |

### B. Intrinsic protein fluorescence of ATH

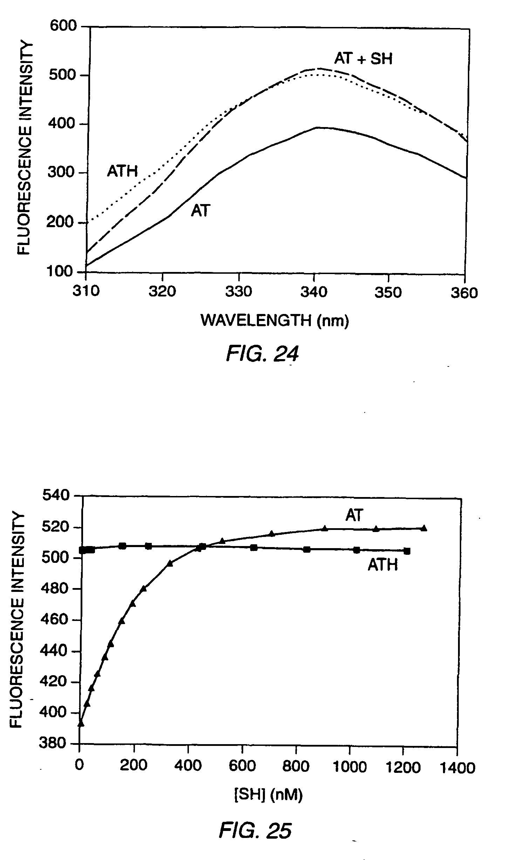

Since heparin is known to induce a ∼ 33 % enhancement in intrinsic protein fluorescence of AT (Huntington *et al* (1996) *Biochemistry* 35,8495-8503), the intrinsic fluorescence of ATH was compared to that of AT and AT + standard heparin (SH). The protein fluorescence emission spectra of 100 nM AT, 100 nM AT plus 1277 nM SH, or 100 nM ATH were recorded (λₑₓ 280 nm, λₑₘ 310-360nm). The fluorescence of AT + H was 32 % higher than that of AT alone at λₘₐₓ (341 nm) with less than a 1 nm peak shift (Figure 24). The spectrum of ATH was virtually identical to that of AT + SH. These data suggest that the conformation of ATH resembles that of the noncovalent AT-SH complex.

### C. Heparin titration of AT and ATH

A titration with SH was performed to determine whether ATH could undergo further conformational change (Figure 25). Protein fluorescence values (at 341 nm) were determined during a SH titration of 100 nM AT and ATH. AT underwent a dose-dependent and saturable increase in fluorescent intensity that yielded a K_{d} of 100 nM and a 32 % maximal ΔFI. In contrast, there was no increase in FI with SH titration of ATH indicating no further alteration in protein conformation. Therefore, ATH is in a fully activated conformation that is independent of exogenous SH.

### D. AT titration of ATH

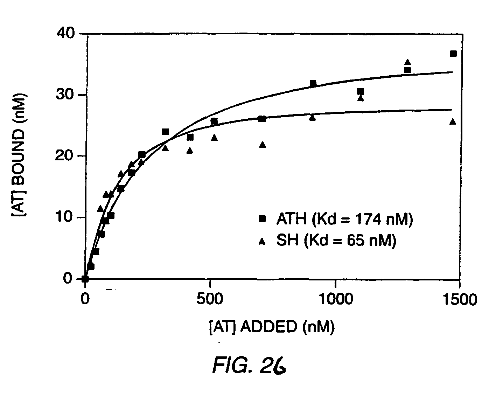

In order to determine whether the heparin component of ATH was capable of binding additional AT, an AT titration of ATH was performed (Figure 26). This was compared to an AT titration of free SH. Protein fluorescence values (341 nm) of 100 nM ATH were determined in the presence of increasing amounts of AT. The values were corrected for inner filter effect such that a control AT titration was linear. The ΔFI values were converted to AT concentration using an extinction coefficient for AT+SH determined in these studies. Binding of AT to SH and to ATH was saturable with K_{d} values of 65 and 175 nM, respectively. The results indicate that there is 28 nM SH bound to AT in 100 nM SH, suggesting ~ 28 % pentasaccharide content in this SH preparation. ATH is able to bind ~ 37 nM AT, revealing a higher pentasaccharide content. These results reveal that the heparin component of ATH is capable of binding additional AT and, therefore, is able to act catalytically.

### E. Protein conformation of ATH compared to AT + H

In a heparin titration, the protein conformation of ATH in the absence of SH, as measured by tryptophan fluorescence, is very similar to that of AT with saturating levels of SH (Figure 25). Therefore, within experimental error, it appears that ATH resembles SH-activated AT. Furthermore, ATH does not undergo further conformational change when SH is added suggesting that no further activation occurs. Therefore, as expected, ATH represents a fully activated form of AT that does not require exogenous SH.

### F. Binding by the H in ATH of additional AT

When AT is added to ATH there is a further increase in protein fluorescence that is due to the intrinsic H within the ATH complex. The K_{d} of binding reveals that the affinity of H (within ATH) for AT is slightly lower than that of free SH (Figure 26). This probably reflects competition between the covalently attached and free AT molecules. The results suggest that about 30 nM AT can bind to 100 nM SH, suggesting a pentasaccharide content of - 30% . Although the ATH -mediated binding to AT revealed higher binding, the apparent pentasaccharide content was only about 1/3 higher (∼ 40 nM binding to AT from 100 nM ATH). This is unexpected but may be due to the competition of the AT in ATH with the exogenous AT, or that 1/3 of ATH molecules have a second pentasaccharide of the AT in ATH with the exogenous AT, or that of ATH molecules have a second pentasaccharide. These results suggest that the H within ATH is catalytic.

### G. Selection for heparin molecules with two pentasaccharides in formation of ATH

When a fixed amount of heparin is titrated with AT and the fluorescence intensity is monitored, there is a saturable increase in fluorescence intensity that reflects heparin-induced conformational changes in the reactive center of AT. A similar increase in fluorescence intensity is observed when ATH is titrated with AT (*see* Figure 9).

The results summarized in Figure 9, reflecting a change in fluorescence intensity that occurs when ATH is titrated with AT which is almost identical to that which occurs when heparin is titrated with AT, suggest the presence of a second pentasaccharide on the AT-conjugated heparin. This can be demonstrated by considering the result that would be expected if heparin having only one pentasaccharide were conjugated to the AT moiety of ATH. In that case, as the pentasaccharide disengaged itself from the AT to which the heparin is covalently bound, the AT would return to its native conformation, resulting in a decrease in fluorescence intensity. Once disengaged, the pentasaccharide could then bind an exogenous AT causing it to undergo a conformational change. This would be associated with a reciprocal increase in fluorescence intensity back to the starting value. The net effect of this process would be no change in fluorescence intensity, contrary to what is observed in this experiment.

### EXAMPLE III

### Production and Purification of ATH

Human AT (Bayer Inc.) and SH (Sigma Chem. Co. U.S.A.) were initially dialysed to ensure purity of the reagents. Human AT and SH were incubated together in a 40°C water bath for 10-14 days. This incubation allowed the conjugation of heparin to AT by Schiff base formation between the aldose terminus aldehyde on heparin and a lysyl amino on the AT, followed by an Amadori rearrangement or reduction by sodium cyanoborohydride (final concentration 0.05M) for 5 h after the initial reaction. The sodium cyanoborohydride was added to the mixture after the incubation period. This production process is simple and does not require any structural changes to either compound.

ATH was purified using two chromatographic steps.

The first step involves adding the reaction mixture to a hydrophobic-containing matrix, butyl-agarose, in 2.5 M ammonium sulphate. Under these conditions, free AT and ATH bind to butyl-agarose beads while heparin does not. AT and ATH are than eluted off the beads by adjusting the ammonium sulphate concentration to less than 1.5 M. The ATH and AT that are eluted off the butyl-agarose matrix are then dialysed against 0.01 M Tris-HCl pH 8.0 buffer.

The second step involves applying the eluted ATH and AT onto DEAE Sepharose Fast Flow Beads in 0.2 M NaCl. Under these conditions, free AT does not bind to the DEAE Sepharose Beads. ATH is then eluted off the DEAE beads by adjusting the NaCl concentration to 2 M. The purified ATH is then concentrated by pressure dialysis at 4°C under 1 atmosphere of nitrogen pressure in tubing with a 12000-14000 molar mass cut-off.

### EXAMPLE IV

### Stability of ATH

ATH was stored at 4°C and anti-factor Xa activity assays were performed on the compound on a regular basis over 3 months. Two anti-factor Xa activity assays were used. The first had no exogenous AT added while, in the second, exogenous AT was added. Table 3 shows that the ATH lost activity after about three months.

ATH has also been stored at -70°C, with no loss of activity after six months. ATH has also been lyophylized and reconstituted with water. Prior to freeze drying, ATH was dialysed against 0.1 M Alanine and 0.15 M NaCl pH 7.0. Reconstituted ATH was active, as assayed by anti-factor Xa activity, for at least 6 months.

**TABLE 3**

| Anti-Factor Xa Activity of ATH Over Time When Stored at 4°C | | | |
|---|---|---|---|
| Date of Assay | Days After Start of Storage at 4°C | Anti-Factor Xa Activity AT Added (u/ml) | Anti-Factor Xa Activity No AT added (u/ml) |
| 23.11.95 | pre | 1630 | not done |
| 11.12.95 | 5 | 1740 | 107 |
| 20.12.95 | 14 | 1760 | not done |
| 23.01.96 | 48 | not done | 62 |
| 13.03.96 | 96 | not done | 0.52 |

### EXAMPLE V

### Biological Activities and Mechanisms of Action of ATH

### 1. Direct non-catalytic activity

ATH has direct non-catalytic antithrombin activity as well as anti-factor Xa activity. Using a standard anti-factor Xa assay (*Thrombosis Res.* 10:399-410 (1977)) without exogenous AT added, ATH has a specific activity of 48 U/mg heparin.

Inhibition of thrombin was studied by measuring the residual activity of thrombin using the chromogenic substrate S2238 (*Thrombosis Res.* 13:285-288 (1978)) after the enzyme had been reacted with ATH. The activity of ATH was compared to AT or AT+SH. The amounts of AT and or heparin used were equivalent by weight to the amounts of each used in the ATH. Figure 10 shows that when the AT and heparin components are present in an equal mass, ATH is much more active than AT+SH in inhibiting thrombin.

### 2. Catalytic activity

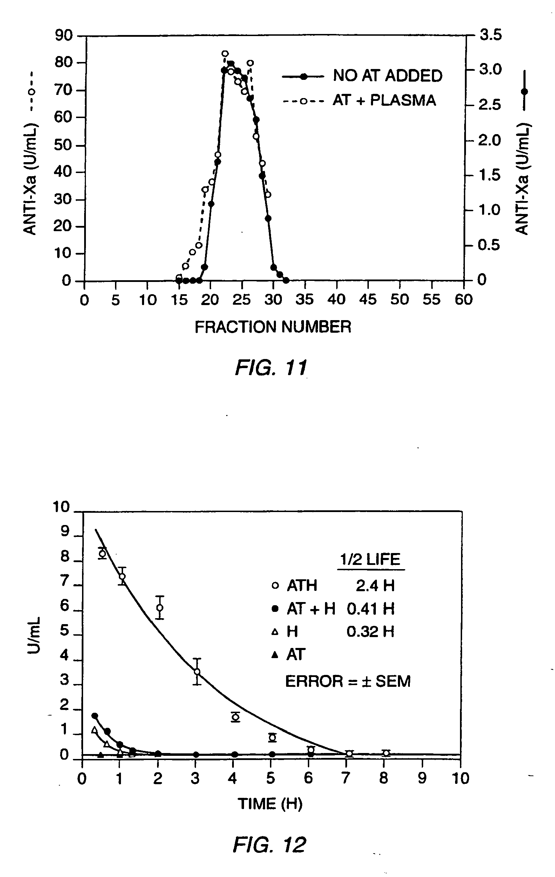

The anti-factor Xa activity of ATH without exogenous AT added to the assay system was 48 u/mg. For an equivalent amount of AT, there was no measurable activity. The antifactor Xa activity of ATH with exogenous AT added to the assay system was 731 u/mg heparin, indicating that there was catalytic activity in the ATH. Since AT is covalently linked to H in the complex, the observation that the H in ATH could catalyze AT mediated inactivation of Xa is unexpected. To rule out the possibility that the observed catalytic effect of the ATH is due to contamination of ATH with free H, ATH was subjected to gel filtration over a G-200 column. The eluted fractions were then analyzed on a 4% stacking and 7.5 % separating polyacrylamide gel which clearly separates ATH from heparin. The heparin in the ATH and in the free heparin fraction was detected by alcian blue staining followed by silver nitrate and the amount of heparin in the ATH and free heparin bands was quantified using densitometry and comparing the weights of paper cut out from the area under the curves. The data are summarized in Table 4 and Figure 11.

A fraction was selected (fraction 22) which contained 0.100818 mg H/ml as ATH and 0.498200 µg H /ml as free heparin and was assayed for anti-factor Xa activity. The specific anti-factor Xa activity of this fraction was 83.25 U/ml. If this was accounted for only by the amount of ATH present in the fraction it would be equivalent to a specific activity of 825 U/mg. If the anti-factor Xa activity in the fraction was accounted for only by the free heparin in the fraction it would require the heparin to have a specific activity of 167101 U/mg. Since the specific anti-factor Xa activity of SH is about 160 U/mg, and the amount of free heparin in the fraction is less than 0.5 µg/ml, the results of this experiment indicate that almost all of the observed anti-factor Xa activity is accounted for by ATH. The specific anti-factor Xa in this fraction (fraction 22) was assayed in the presence and absence of exogenous AT. The activity was increased 25 to 30 fold in the presence of exogenous AT. Based on the very low concentration of free heparin (described above), this fold increase could only be explained by a catalytic effect of heparin in the ATH complex. To verify this point, anti-factor Xa assays were performed in the presence of exogenous AT using heparin (high affinity) in concentration of 0.5 (the amount of free heparin in the fraction) and 5 µg/ml. In both cases, there was no measurable anti-factor Xa activity. These findings indicate that the catalytic activity observed in the ATH could not be due to contaminating free heparin, and confirm that complexed heparin in ATH has catalytic activity. The ratio of catalytic to non catalytic activity was significantly greater in high molecular weight fractions compared to low molecular weight fractions. This suggests that a higher number of pentasaccharides (*i*.*e*., two or more pentasaccharides per molecule) are present in larger ATH molecules.

**Table 4**

| Anti-Factor Xa Activity of Gel Filtered ATH | | | |
|---|---|---|---|
| Fraction | Anti-Factor Xa Activity (u/ml) | Specific Activity of ATH if the Activity Was Due to ATH Alone (u/mg heparin) | Specific Activity of Free Heparin If the Activity Was Due to Free Heparin Alone (u/mg heparin) |
| 22 | 83.25 | 825.88 | 167101.6 |
| 24 | 73.26 | 634.10 | 34410.5 |
| 26 | 79.92 | 817.10 | 14468.3 |
| 28 | 43.29 | 658.10 | 4234.6 |
| 30 | 33.00 | 1005.3 | 3141.2 |

Without intending to be bound by any particular mechanism, there are two likely explanations for the observed catalytic effect of ATH.

The less likely is that when the AT component of ATH complex binds to thrombin, a conformational change occurs at the heparin binding site of AT, which results in a markedly reduced affinity for the heparin pentasaccharide. The pentasaccharide then dissociates from the AT (although the heparin molecule remains covalently linked to the AT) and is available to bind to exogenous AT.

More likely is the possibility that the process of covalent linkage of AT to heparin selects heparin molecules that contain two pentasaccharide units. Therefore, ATH can bind to AT and acts as a catalyst through the second pentasaccharide site.

In order to clarify the mechanisms responsible for the observed catalytic activity, the following experiments can be performed:
i) To differentiate between the two mechanisms, ATH will be passed over an AT column. If ATH binds to immobilized AT, it would imply that the second mechanisms is responsible. In addition the anti-factor Xa activity of ATH would be expected to be decreased by heparinase treatment if a second pentasaccharide is responsible for the increased activity.
ii) If ATH does not bind to immobilized AT it would support the first suggested mechanism as the cause of the observed catalytic effect of heparin covalently bound to AT. To evaluate this mechanism, ATH will be titrated with thrombin before passing it over an AT column. Active site-inhibited thrombin (FPR-thrombin) will be used as a control, since this does not bind to the reactive center of AT and would therefore not be expected to reduce the affinity of AT to the pentasaccharide.

### 3. Inactivation of ATH by Protamine

The ability of protamine sulphate and of human platelet factor 4 (PF4) to inactivate the anticoagulant activity of ATH was determined. About 80% of the anti-factor Xa activity is inactivated by either protamine sulphate or PF4. Thus, ATH activity can be neutralized during use, if necessary.

### 4. Rate of Inhibition of thrombin

The second order rate constants of ATH, AT alone and AT+SH, were compared, using the method of Hoyiaerts *et al.* (*J. Biol. Chem.* 259(9):5670-5677). As shown in Table 5, ATH is about 30 times faster than AT+SH at inhibiting thrombin.

**Table 5**

| Second Order Rate Constants for ATH | | |
|---|---|---|
| | Second Order Rate Constants (M⁻¹s⁻¹) | Fold Increase Over AT Alone |
| AT | 7.0 x 10³ | |
| AT+SH (saturating in AT) | 1 x 10⁸ | 14000 |
| ATH | 3.1 x 10⁹ | 440,000 |

### 5. Effect of Fibrin on Thrombin Inactivation by ATH

Thrombin bound to fibrin remains catalytically active, dissociates very slowly from fibrin, and is protected from inactivation by AT and by AT and SH. The effect of ATH on fibrin bound thrombin was evaluated and the apparent k1 of the rate of thrombin inhibition by ATH in the presence of different concentrations of fibrin monomer determined by measurement of residual thrombin at various times during the reaction. Inhibition of thrombin is stopped at various times by addition of polybrene and the thrombin activity remaining is determined using the chromogenic substrate S-2238. The results are presented in Table 6. The rate of thrombin inhibition by ATH was unaffected by fibrin monomer. In contrast, fibrin monomer decreased the ability of high affinity heparin to inhibit thrombin by about 60 fold. These results indicate that ATH can inactivate thrombin bound to fibrin.

**Table 6**

| Comparison of Effect of Firbin in Monomer on Rate of Thrombin Inhibition by 100 nM ATH verses 100nM H plus 200 mM AT | | |
|---|---|---|
| Fibrin monomer concentration (uM) | Fold inhibition | |
| | ATH | AT + H* |
| 0.0 | 1.00 | 1.0 |
| 0.5 | 1.03 | 30.0 |
| 1.0 | 1.23 | 38.0 |
| 4.0 | 1.24 | 58.1 |

| | | |
|---|---|---|
| * High affinity heparin. High affinity heparin is the ATIII binding fraction of heparin purified from standard SH. | | |

Fibrin-bound thrombin is resistant to inactivation by SH because the heparin binding site (exosite 2) on thrombin is masked when the enzyme is bound to fibrin. Since ATH can inactivate fibrin bound thrombin, experiments were preformed to determine whether exosite 2 is critical for inactivation of thrombin by ATH. These experiments were carried out using R93-thrombin, a recombinant thrombin with an inactive mutant exosite 2 (*J. Biol*. *Chem*. 269:17965-17970 (1994)). As shown in Table 7, ATH inactivates R93-thrombin at the same apparent rate as alpha thrombin. In contrast to ATH, the kl of high affinity heparin is about 400 times higher for alpha thrombin than for R93-thrombin. These findings suggest that exosite 2 is not required for ATH to bind to thrombin.

**Table 7**

| Rate of Thrombin (IIa) Inhibition By ATH vs High Affinity Heparin (HASH) | | | |
|---|---|---|---|
| Type of Thrombin | Inhibitors Used | k1 (1/min) (apparent for ATH, corrected for HASH+AT) | k1(alpha IIa)/k1(R93 IIa) |
| alpha IIa | ATH | 3.45 | 1.0 |
| R93 IIa | ATH | 4.19 | 1.2 |
| alpha IIa | HASH+AT | 63.79 | 1.0 |
| r93 IIa | HASH+AT | 0.14 | 0.002 |

### EXAMPLE VI

### Pharmacokinetic Studies of ATH in Rabbits

The pharmacokinetics of ATH was studied in rabbits using anti-factor Xa assays and ELISAs for human AT. Pharmacokinetics of human AT+SH, SH alone and human AT alone in rabbits were studied for comparison with ATH.

### 1. Pharmacokinetics after Intravenous Administration in Rabbits

The amounts of each compound administered intravenously to rabbits were as described below. Anti-factor Xa activity was assayed by the method described in *Thrombosis Res.* 10:399-410 (1977).

| | | AT Given | Heparin Given | Anti-Factor Xa Activity Given |
|---|---|---|---|---|
| 1. | ATH | 2.75 mg/kg | 0.698 mg/kg | 544.3 u/kg |
| 2. | AT + SH | 2.75 mg/kg | 0.698 mg/kg | 124.8 u/kg |
| 3. | SH | | 0.698 mg/kg | 124.8 u/kg |
| 4. | AT | 2.75 mg/kg | | |

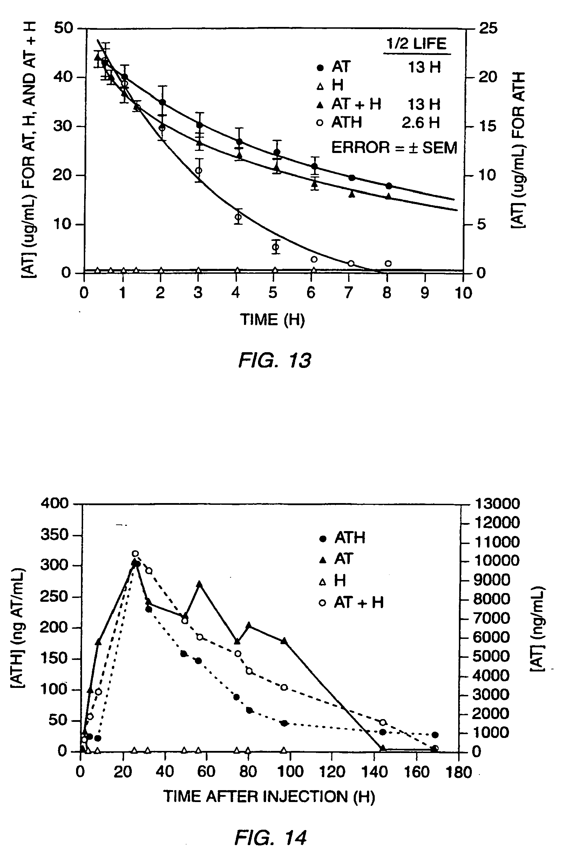

Five rabbits were used for each group. The compounds were administered intravenously to conscious, pathogen free, NZW rabbits. Citrated blood samples were taken from the rabbits at different time points up to 24 hours. Anti-factor Xa assays and ELISAs of human AT were performed on each sample. The half lives of ATH, AT+SH and SH by anti-factor Xa activity are about 2.4 hours, 0.41 hour and 0.32 hour respectively. The half lives of ATH, AT+SH and AT by ELISAs of human AT are 2.4 hours, 13 hours and 13 hours respectively. The results are summarized in Figures 12 and 13 and Table 8. The half lives of SH after intravenous injection and AT in humans are reported to be about 60 minutes and 66 hours respectively, which is approximately 2 times the half life of SH and 5 times the half life of AT in rabbits. Based on these observations, the half life of ATH in humans is expected to be 2-5 times that in the rabbit, which is about 5 hours to 12 hours. This long half life of ATH will be a distinct advantage for use in prophylaxis, as it can be administered infrequently. The maximal anti-factor Xa activities for ATH and SH were 8.4 u/ml and 1.17 u/ml respectively.

**Table 8**

| Half Life of ATH in Rabbits | | | |
|---|---|---|---|
| | t½ by Anti-Factor Xa In Rabbits | t½ by ELISA in Human AT in Rabbits | t½ Reported in Human |
| ATH | 2.4 hours | 2.6 hours | |
| AT + SH | 0.41 hours | 13 hours | |
| SH | 0.32 hours | | 1 hour |
| AT | | 13 hours | 66 hours |

### 2. Pharmacokinetics after Subcutaneous Administration in Rabbits

The amounts of compound administered subcutaneously to the rabbits were as follows:

| | | AT Given | Heparin Given | Anti-Factor Xa Activity Given |
|---|---|---|---|---|
| 1. | ATH | 4.6 mg/kg | 1.2 mg/kg | 936 u/kg |
| | | 5.4 mg/kg | 1.7 mg/kg | 1325 u/kg |
| 2. | AT + SH | 4.6 mg/kg | 1.2 mg/kg | 216 u/kg |
| | | 5.4 mg/kg | 1.7 mg/kg | 306 ug/kg |
| 3. | SH | | 1.2 mg/kg | 216 u/kg |
| | | | 1.7 mg/kg | 306 u/kg |
| 4. | AT | 4.6 mg/kg | | |
| | | 5.4 mg/kg | | |

Two dosages were tested and one animal was used for each dose. The compounds were administered subcutaneously to conscious, pathogen free, NZW rabbits. Citrated blood samples were taken from the rabbits at different time points up to 170 hours. Anti-factor Xa assays and ELISAs of human AT were performed on each sample. The maximal antifactor Xa activity for SH was 0.29 u/ml at 1 hour but there was essentially no anti-factor Xa activity in rabbits that received ATH. Figure 14 shows the mean concentration of AT over time. These results suggested that ATH was not absorbed well with the dosage given by the subcutaneous route. This is probably due to the size of the molecule.

### 3. Pharmacokinetics after Tracheal Instillation in Rabbits

One potential use for ATH is to treat respiratory distress syndrome. Therefore, the effect of ATH after tracheal instillation was investigated.

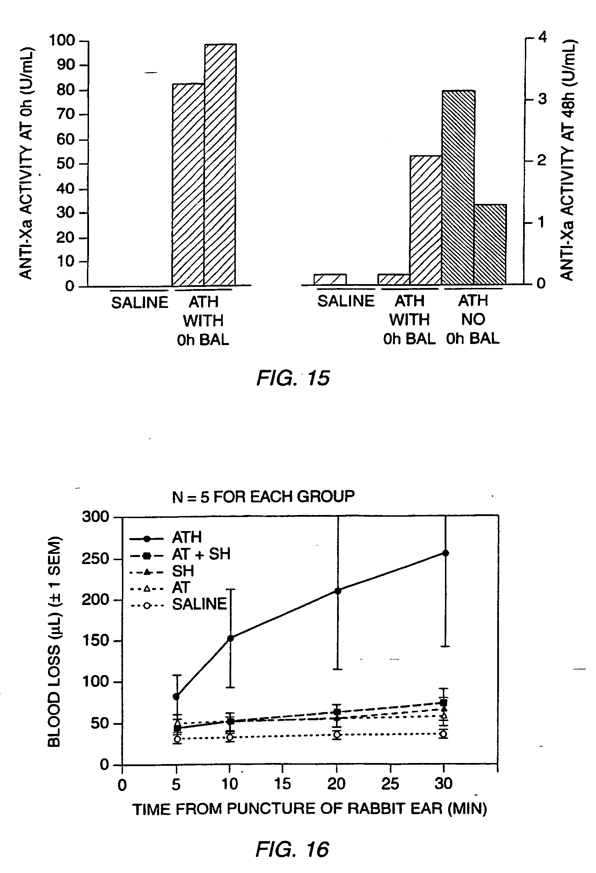

ATH and saline were administered intratracheally through an endotracheal tube to anaesthetized pathogen free NZW rabbits. The amount of ATH administered was 100 anti-factor Xa u/kg. Four rabbits were used for ATH and two rabbits used for saline. For two of the rabbits that received ATH and rabbits that received saline, bronchoalveolar lavage (BAL) was done immediately after the instillation to assess whether it is possible to remove the compound after administration. BAL were collected on all animals at 48 hours. Citrated blood samples were taken at multiple time points up to 48 hours. Anti-factor Xa assays were done on both BAL and blood samples. There was essentially no anti-factor Xa activity in the blood samples. For the BAL at the time 0 hour, a significant amount of ATH was removed as evidenced by the high anti-factor Xa activity. At 48 hours, there was still anti-factor Xa activity remaining in the BAL (Figure 15). These preliminary results demonstrated that ATH remained in the lung for a prolonged period of time and did not give rise to a significant anticoagulant effect systemically.

### EXAMPLE VII

### Antithrombotic and Haemorrhagic Effects of ATH in Experimental Models: Comparison with Heparin

The safety and efficacy of ATH has been tested in two animal models. The results of these experiments demonstrate that (i) ATH prevents thrombus growth and accelerates physiologic fibrinolysis in an animal model of venous thrombosis, and (ii) ATH is effective at doses that have acceptable haemorrhagic effects.

### 1. Comparison of ATH with Heparin in a Rabbit Bleeding Model

We compared the relative effect of ATH, AT+SH, SH alone, AT alone and saline on experimental bleeding using a rabbit bleeding ear model. The 5 treatment arms included:

| | | AT Given | Heparin Given | Anti-Factor Xa Activity Given |
|---|---|---|---|---|
| 1. | ATH | 1.10 mg/kg | 0.279 mg/kg | 217.7 u/kg |
| 2. | AT+SH | 1.10 mg/kg | 0.279 mg/kg | 49.9 u/kg |
| 3. | SH | - | 0.279 mg/kg | 49.9 u/kg |
| 4. | AT | 1.10 mg/kg | - | - |
| 5. | Saline | - | - | - |

The doses given were equivalent by weight. Five rabbits were studied in each group.

In these experiments, rabbits were anaesthetized and test compounds were given as an intravenous bolus. Five minutes after the compounds were injected, one ear of the rabbit was punctured by a #11 surgical blade five times in a random fashion avoiding areas with visible vessels. The ear was then placed in a 37°C water bath (total volume of 1 litre) that was stirred continuously. Ten ml aqueous samples from the water bath were taken at 5 minutes, 10 minutes, 20 minutes and 30 minutes from the time of the ear being punctured. Citrated blood samples were also taken at the same time points. Samples were centrifuged immediately at 1,700 g, platelet-poor plasma obtained and frozen at -70°C until assays were performed.

Anti-factor Xa assays were done on the plasma samples. Absorbance of the water samples were measured at a wavelength of 540 nm and results were compared to a standard curve of known amounts of blood in water and the accumulative blood loss over time was calculated.

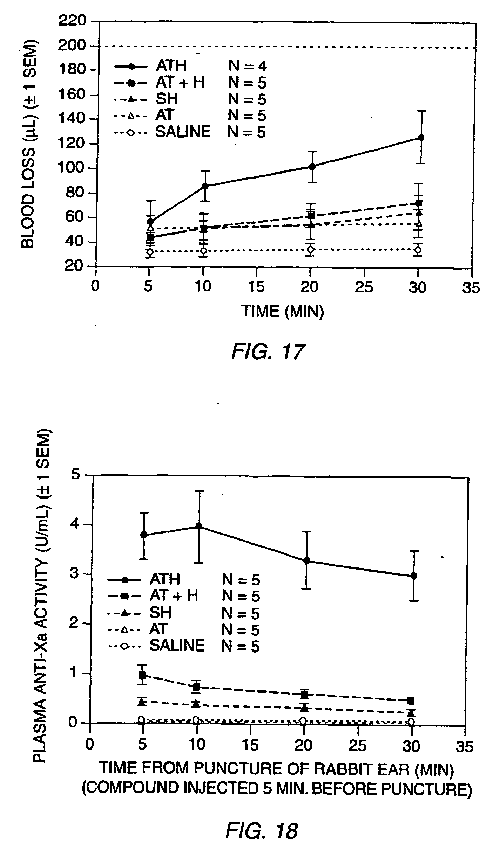

Figure 16 shows the cumulative blood loss over time. Bleeding was highest in the ATH group. One animal in the ATH group had significantly more bleeding than the rest of the animals in the same group. Figure 17 shows the cumulative blood loss over time when this outlier was taken out of the analysis. The bleeding from the animals in the ATH group was well below the accepted amount of 200 pi blood loss over 30 minutes. Moreover, the cumulative blood loss in the first five minutes was essentially the same for all treatment groups that had anticoagulant. The increased cumulative blood loss in the ATH group is then likely due to its prolonged anti-factor Xa activity. The increased bleeding from ATH may also reflect the fact that anti-factor Xa activities were four times greater than those in the group that received AT+SH. Figure 18 shows the plasma anti-factor Xa activity over time and demonstrates that anti-factor Xa activities of ATH last longer compared to the group that received AT+SH.

### 2. Comparison of ATH with Heparin in a Rabbit Venous Thrombosis Model

We evaluated ATH in a rabbit venous thrombosis treatment model. In these experiments ATH was compared to AT+SH, SH alone, AT alone and saline. The doses used were the same as those used for the rabbit bleeding ear model. The number of rabbits used for each group were n = 5 for ATH, n = 7 for AT+SH, n = 8 for SH, n = 5 for AT and n = 5 for saline.

The rabbits were anaesthetized. The jugular vein was isolated and the side branches over 2 cm of the jugular vein ligated. The jugular vein segment was isolated with 2 tourniquets and a fogartry catheter inserted into the segment of vein. The endothelium was denuded by 15 passes of the inflated catheter and then 500 u of thrombin was injected into the segment. Then 0.2 ml of the rabbit's blood was injected into the segment to create a thrombus. At the same time 0.2 ml of the blood was placed into each of the two test tubes, acting as a control for the weight of the clot. Thirty minutes after the blood was injected into the vein, the tourniquets were released and the blood clot was exposed to systemic circulation. Ten minutes prior to the release of the tourniquets, the compounds tested were injected into the animals followed immediately by an injection of 125 I-human fibrinogen. A 2 ml citrated blood sample and 1 ml clotted blood sample were taken at 10, 20, 30, 60, 120 and 180 minutes after the tourniquets were released. The citrated blood samples were centrifuged to obtain platelet poor plasma and then stored at -70°C. These samples were subsequently assayed for anti-factor Xa activity. At 180 minutes, the animals were euthanised and thrombi recovered. The weight and radioactivity of the thrombi were compared with the control thrombi from the same animal.

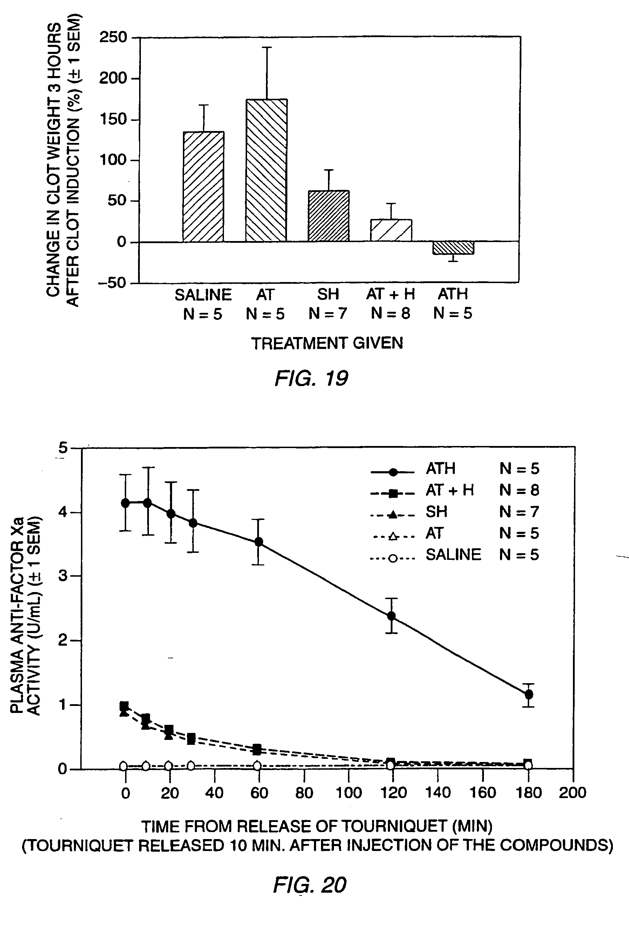

The model is designed to test the ability of an anticoagulant to prevent thrombus growth. The results shown in Figure 19 indicate that ATH, AT+SH and SH were more effective than the saline control and AT control in preventing thrombus growth. However, ATH was the most effective treatment and was associated with an 18 % reduction in thrombus size. The decrease in clot size was similar to results when agents that have activity towards fibrin bound thrombin were used. These data suggest that ATH has activity against fibrin bound thrombin. However, Figure 20 shows that rabbits which received ATH had a higher anti-factor Xa activity compared to other groups. Therefore, it remains to be tested whether the more efficacious effect of ATH is due to a higher anti-factor Xa activity or to an accelerated activity of ATH itself.

It is likely that equivalent anti-factor Xa activities of heparin and ATH will result in less bleeding with ATH and that the reduced bleeding with ATH may be due to limited antiplatelet activity.

### EXAMPLE VIII

### ATH as a Local Anticoagulant to Coat a Prosthetic Surface

ATH was used as a local anticoagulant to coat thrombogenic prosthetic surfaces. To do this, a polyurethane-polycarbonate endovascular tubing from Corvita was coated with ATH by covalent linkage of the urethane groups to ATH by an intermediate monomer linker. The thrombogenecity of the coated tubing was tested in a Rabbit Jugular Vein Model (rabbit perfusion model), and compared to hirudin coated tubing, AT coated tubing and non-treated tubing.

### 1. Methods of Coating Polyurethane-polycarbonate with ATH

Three steps are involved in the chemistry for coating ATH onto polyurethane-polycarbonate. First, the polymer of polyurethane-polycarbonate is activated with NaOCl. NaOCl reacts with urethane to make this relatively inert material chemically reactive. Second, a linking monomer (allyl glycidyl ether) is grafted onto the surface by reacting the activated tubing with an indicator (Na₂S₂O₄) and a monomer that can further react with other compounds such as ATH. Third, ATH (or other anticoagulants that have groups, such as, an amino group, that can react with the functional group of the monomer) is linked to the monomer.

### 2. Comparison of ATH coated tubing with Hirudin coated tubing

Hirudin was linked to polyurethane-polycarbonate tubing using the same method as that used for linkage of ATH. In these experiments, New Zealand White male rabbits were anaesthetized. The femoral artery and vein were cannulated with a cannula used for fluid administration and blood collection. The external jugular vein was exposed and a small segment of the facial vein partially occluded. A modified 14 gauge Angiocath (5 cm long) was inserted into the jugular vein. A 2 cm segment of the endovascular tubing was weighed and inserted into a modified 14 gauge Angiocath (5 cm long) catheter. The modification of the Angiocath consisted of cutting the tip off its stylet. The catheter was inserted 5 cm into the jugular vein via the partially occluded facial vein and the tubing then deployed. Thereafter, the catheter was withdrawn and the facial vein segment ligated. The tubing location can be seen through the jugular vein wall. Prior to insertion of the tubing and at 60, 120, 180 minutes after its deployment, 1 ml of blood was collected into citrate-PPACK as well as into citrate-THAT-M for thrombin-antithrombin complex (TAT) and fibrinopeptide A (FPA) analysis. At the end of 180 minutes, the segment of the external jugular vein containing the tubing was removed, flushed with 10 ml of saline and the outside diameter measured using callipers. Thereafter the segment of vein containing the tubing was opened longitudinally with scissors and the vein peeled off from the tubing. The tubing was cut longitudinally into two halves, blotted slightly on gauze and weighed. Blood samples were centrifuged immediately at 1,700 g, platelet-poor plasma obtained and frozen at -70°C until assays were performed. The tubing were stored in 10% formalin for histopathology.

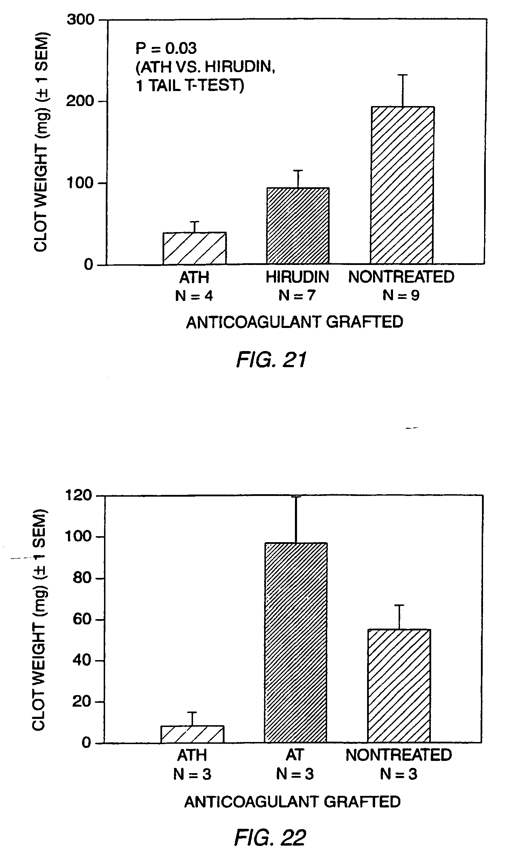

Figure 21 shows the weight of clots formed inside the tubing after they were inserted into rabbits for three hours. As shown in the graph, the weight of clots formed within the ATH coated tubing was statistically and strikingly less than that in the hirudin coated tubing, demonstrating that ATH coated tubing is more effective than Hirudin coated tubing

### 3. Comparison of ATH coated tubing with AT coated tubing and non-treated tubing

The experimental procedures were the same as above. Figure 22 shows the weight of clots that were formed inside tubing after the insertion into rabbits for three hours. ATH coated tubing induced smaller clots than AT coated tubing and non-treated tubing. Thus, the AT coated tubing was significantly more thrombogenic than ATH coated tubing.

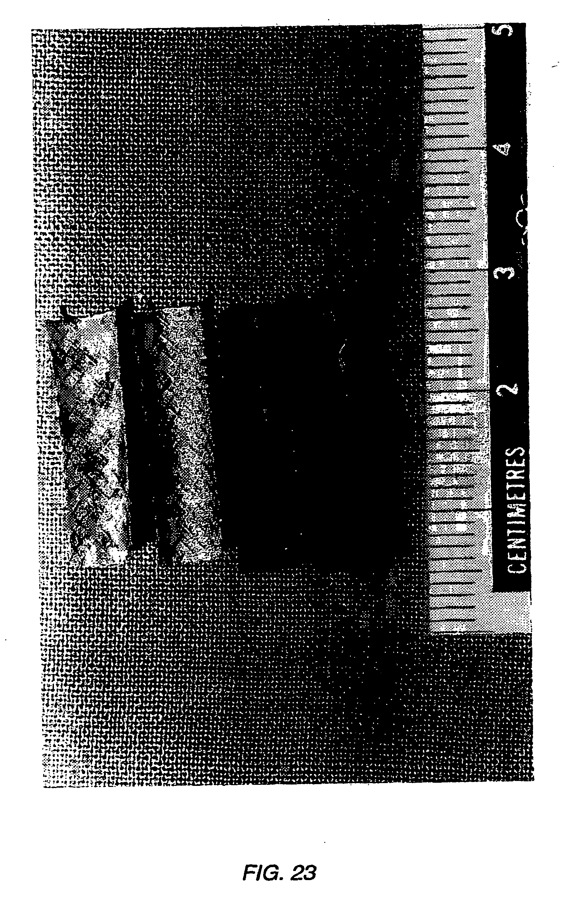

Figure 23 shows the luminal surface of an ATH coated tubing and a non-treated tubing after exposed to blood in a rabbit for three hours. The ATH coated tubing had minimal amount of blood clot on the surface but the non-treated tubing had clearly induced more clot.

## Claims

1. A covalent conjugate comprising a glycosaminoglycan linked to a protein by a covalent linkage wherein said protein comprises at least one primary amino group, wherein said protein is directly covalently linked via said amino group to a terminal aldose residue of said glycosaminoglycan, said covalent linkage comprising an α-carbonyl amine formed by subsequent Amadori rearrangement, for a sufficient period of time, of an imine resulting from reaction between said amino group and said terminal aldose residue of said glycoaminoglycan and pharmaceutically acceptable salts thereof, wherein said glycosaminoglycan is heparin.

2. The conjugate of claim 1, wherein said covalent linkage comprises a -CO-CH₂-NH- group formed by Amadori rearrangement of a -HCOH-HC=N- group resulting from reaction between said amino group and the C1 carbonyl group of said terminal aldose residue.

3. The conjugate of claim 2, wherein within the -CO-CH₂-NH- group, the -CO-CH₂ portion is derived from the glycosaminoglycan and the -NH- portion is derived from the amino group of the protein.

4. A conjugate which has the formula: glycosaminoglycan-CO-CH₂-NH-protein, wherein the glycosaminoglycan is heparin and the protein is antithrombin III (AT).

5. The conjugate according to any preceding claim, wherein the molar ratio of said protein to glycosaminoglycan is less than one.

6. The conjugate according to any of claims 1 to 5, wherein the molar ratio of said protein to glycosaminoglycan is 1:1.

7. The conjugate of any preceding claim, wherein said protein is a serine protease inhibitor.

8. The conjugate of claim 7, wherein said protease inhibitor is selected from the group consisting of antithrombin III and heparin cofactor II.

9. The conjugate of any preceding claim, wherein the glycosaminoglycan is heparin, and the protein is antithrombin III.

10. A conjugate according to claim 9, which is more effective at inhibiting thrombin than free antithrombin-III and heparin.

11. A conjugate according to claim 9, having a molecular weight of 69kD-100KD.

12. A conjugate according to claim 9, 10 or 11 which possesses greater than 60%, 90%, 95% or 98% the antithrombin activity of intact unconjugated heparin.

13. A conjugate according to claim 9, which has a longer half life than heparin *in vivo.*

14. The conjugate of any preceding claim, wherein the glycosaminoglycan is heparin and the heparin comprises a single pentasaccharide.

15. The conjugate of any preceding claim, wherein the glycosaminoglycan is heparin and the heparin comprises two pentasaccharides.

16. A conjugate of claim 15, including more than 10% heparin chains having two pentasaccharides.

17. A conjugate according to any preceding claim, pharmaceutically suitable for injection in a human.

18. A one step process for conjugating a protein comprising at least one primary amino group to a glycosaminoglycan, said process comprising incubating said glycosaminoglycan with said protein under conditions which allow formation of an imine between said amino group and a terminal aldose residue of said glycosaminoglycan, wherein the glycosaminoglycan is heparin, and wherein the imine is rearranged to an α-carbonyl amine.

19. A process according to claim 18, wherein said α-carbonyl amine is formed by Amadori rearrangement of the imine.

20. A process according to claim 18, which comprises incubating said glycosaminoglycan with said protein at a pH suitable for imine formation between the said amino group and the terminal aldose residue of the glycosaminoglycan and for a time sufficient to allow Amadori rearrangement of said imine to an α-carbonyl amine.

21. A process as claimed in any of claims 18 to 20, wherein the glycosaminoglycan is heparin and the protein is antithrombin III (AT III).

22. A process for producing a covalent conjugate according to any of claims 1 to 17, which comprises the step of conjugating a protein comprising at least one primary amino group to a glycosaminoglycan by a process according to any of claims 18 to 21.

23. A process according to claim 24, which also comprises the step of isolating the covalent conjugate.

24. A process for preparing a conjugate composition of proteins comprising at least one primary amino group linked to glycosaminoglycans by covalent bonds, said process comprising:
(a) incubating said glycosaminoglycans with said proteins at a pH and for a time sufficient for formation of an imine between said amino group and a terminal aldose residue of said glycosaminoglycans, and at a time and temperature sufficient for said imines to undergo subsequent Amadori rearrangement, for a sufficient period of time, to an α-carbonyl amine forming said covalent bonds; and
(b) isolating said conjugate composition or a pharmaceutically acceptable salt thereof, wherein said glycosaminoglycans are heparin (H) and said amino-containing molecules are antithrombin III (ATIII).

25. A process according to claim 20 or 24, wherein said incubating is carried out from about 3 days to two weeks at a temperature of 35°C to 45°C.

26. A process according to claim 25, wherein said incubating is carried out for about two weeks.

27. A pharmaceutical preparation containing a conjugate according to any of claims 1 to 17 as active ingredient in association with a pharmaceutically acceptable carrier.

28. A pharmaceutical preparation as described in claim 27, in the form of an aqueous solution for injection or in the form of an ointment or in the form of an aerosol.

29. The use of a conjugate according to any of claims 1 to 17, in the manufacture of a medicament for the prophylactic and therapeutic treatment of neonatal respiratory distress syndrome, adult respiratory distress syndrome, primary carcinoma of the lung, non-Hodgkins lymphoma, fibrosing alveolitis, lung transplants, L-asparaginase induced deficiency, cardiopulmonary bypass induced deficiency, sepsis or congenital ATIII deficient states.

30. The use as described in claim 29, wherein said mammal is human.

31. The use of a conjugate according to any of claims 1 to 17, in the manufacture of a medicament for the prevention and treatment of thrombosis.

32. The use of a conjugate according to any of claims 1 to 17, in the manufacture of a medicament for the treatment of infant or adult respiratory distress syndrome.

33. The use as in claim 32, wherein the conjugate of claim 9 is delivered directly to the airways of the lung.

34. The use as in claim 33, wherein a lung surfactant, an anti-inflammatory steroid, an anti-asthmatic drug, or a bronchodilator is concurrently administered.

35. The use of a conjugate according to any of claims 1 to 17, in the manufacture of a medicament for the prevention of fibrin deposition in the lung alveoli.

36. A material for use in a medical or prosthetic device, said material comprising a polymer in contact with a covalent conjugate according to any of claims 1 to 17.

37. The material of claim 36, wherein the glycosaminoglycan is heparin and the species is antithrombin III.

38. The material of claim 36 or 37, wherein the conjugate is covalently attached to the polymer.

39. A material for use in a medical or prosthetic device, said material comprising a polymer in contact with a covalent conjugate composition, said covalent conjugate composition comprising glycosaminoglycans linked to a protein by covalent linkages, wherein the protein comprises at least one primary amino group, wherein the glycosaminoglycans are heparins, the protein is antithrombin III, and the covalent conjugate composition comprises antithrombin III-heparin (ATH), said ATH being covalently attached to the polymer, and wherein the protein is directly covalently linked via said amino group to a terminal aldose residue of the glycosaminoglycans by an α-carbonyl amine linkage.

40. The material of any of claims 36 to 39, wherein the polymer is a synthetic polymer selected from the group consisting of poly 2-hydroxyethyl methoacrylate, poly acrylamide, poly ether polyurethane urea (PEUU), polyethylene, polypropylene, polytetrafluoroethylene, poly (vinylchloride), polydimethylsiloxane, an ethylene-acrylic acid copolymer, Dacron, polyester-polyurethane, polyurethane, polycarbonate-polyurethane, polyamide (Nylon) and polystyrene.

41. The material of claim 40, wherein the polymer is polyurethane-polycarbonate.

42. A prosthetic or medical device comprising the material of any of claims 36 to 41.

43. The device of claim 42, selected from the group consisting of endovascular tubing, an arterial or central venous line, a cardiac catheter, a cardiopulmonary bypass circuit, a dialysis circuit or other external blood contacting instrument, a pacemaker lead, an arterial or venous catheter, a thrombectomy catheter, a suture, a blood filter, an intravenous line, a mechanical valve, a stent, an artificial kidney, lung, heart or liver, and an *in vivo* prosthesis.

44. The device of claim 43, wherein the device is endovascular tubing.

45. A method of reducing the thrombogenicity of a material comprising coating the material with a conjugate according to any of claims 9 to 13.

46. The method of claim 45, wherein the conjugate is covalently bound to the material.

47. The method of claim 45 or 46, wherein the material is suitable for use in a prosthetic or medical device, as defined in claim 43.

48. The use of a conjugate according to any one of claims 1 to 17 to reduce the thrombogenicity of internal and extracorporal devices that contact blood.

## Patentansprüche

1. Kovalentes Konjugat umfassend ein Glycosaminoglycan, das über eine kovalente Verknüpfung mit einem Protein verknüpft ist, wobei das Protein wenigstens eine primäre Aminogruppe umfaßt, wobei das Protein über diese Aminogruppe mit einem terminalen Aldoserest des Glycosaminoglycans unmittelbar kovalent verknüpft ist, wobei die kovalente Verknüpfung ein α-Carbonylamin umfaßt, das ausgebildet wird durch anschließende Amadori-Umlagerung über einen ausreichenden Zeitraum eines Imins, das hervorgeht aus der Reaktion zwischen der Aminogruppe und dem terminalen Aldoserest des Glycosaminoglycans, und pharmazeutisch verträgliche Salze davon, wobei das Glycosaminoglycan Heparin ist.

2. Konjugat nach Anspruch 1, wobei die kovalente Verknüpfung eine -CO-CH₂-NH-Gruppe umfaßt, die ausgebildet wird durch Amadori-Umlagerung einer -HCOH-HC=N-Gruppe, die hervorgeht aus der Reaktion zwischen der Aminogruppe und der C1-Carbonylgruppe des terminalen Aldoserests.

3. Konjugat nach Anspruch 2, wobei in der -CO-CH₂-NH-Gruppe der -CO-CH₂-Anteil von dem Glycosaminoglycan abgeleitet wird und der -NH-Anteil von der Aminogruppe des Proteins abgeleitet wird.

4. Konjugat, welches die Formel: Glycosaminoglycan-CO-CH₂-NH-Protein aufweist, wobei das Glycosaminoglycan Heparin ist und das Protein Antithrombin III (AT) ist.

5. Konjugat nach einem der vorangehenden Ansprüche, wobei das molare Verhältnis des Proteins zu dem Glycosaminoglycan weniger als 1 beträgt.

6. Konjugat nach einem der Ansprüche 1 bis 5, wobei das molare Verhältnis des Proteins zu dem Glycosaminoglycan 1:1 beträgt.

7. Konjugat nach einem der vorangehenden Ansprüche, wobei das Protein ein Serinproteaseinhibitor ist.

8. Konjugat nach Anspruch 7, wobei der Proteaseinhibitor ausgewählt ist unter Antithrombin III und Heparin-Cofaktor II.

9. Konjugat nach einem der vorangehenden Ansprüche, wobei das Glycosaminoglycan Heparin ist und das Protein Antithrombin III ist.

10. Konjugat nach Anspruch 9, welches bei der Hemmung von Thrombin wirksamer ist als freies Antithrombin-III und Heparin.

11. Konjugat nach Anspruch 9, welches ein Molekulargewicht von 69kD-100kD aufweist.

12. Konjugat nach Anspruch 9, 10 oder 11, welches mehr als 60%, 90%, 95% oder 98% der Antithrombin-Aktivität von intaktem, nicht konjugiertem Heparin aufweist.

13. Konjugat nach Anspruch 9, welches eine längere Halbwertzeit als Heparin *in vivo* aufweist.

14. Konjugat nach einem der vorangehenden Ansprüche, wobei das Glycosaminoglycan Heparin ist und das Heparin ein einzelnes Pentasaccharid umfaßt.

15. Konjugat nach einem der vorangehenden Ansprüche, wobei das Glycosaminoglycan Heparin ist und das Heparin zwei Pentasaccharide umfaßt.

16. Konjugat nach Anspruch 15, das mehr als 10% Heparinketten aufweist, die zwei Pentasaccharide aufweisen.

17. Konjugat nach einem der vorangehenden Ansprüche, welches für die Injektion in einen Menschen pharmazeutisch geeignet ist.

18. Einstufiges Verfahren zur Konjugierung eines Proteins, welches wenigstens eine primäre Aminogruppe umfaßt, mit einem Glycosaminoglycan, wobei man bei dem Verfahren das Glycosaminoglycan mit dem Protein unter Bedingungen, die die Bildung eines Imins zwischen der Aminogruppe und einem terminalen Aldoserest des Glycosaminoglycans ermöglichen, inkubiert, wobei das Glycosaminoglycan Heparin ist, und wobei das Imin in ein α-Carbonylamin umgeändert wird.

19. Verfahren nach Anspruch 18, wobei das α-Carbonylamin durch Amadori-Umlagerung des Imins ausgebildet wird.

20. Verfahren nach Anspruch 18, bei dem man das Glycosaminoglycan mit dem Protein bei einem pH-Wert inkubiert, der für die Iminausbildung zwischen der Aminogruppe und dem terminalen Aldoserest des Glycosaminoglycans geeignet ist, und über einen Zeitraum, der ausreichend ist, um die Amadori-Umlagerung des Imins in ein α-Carbonylamin zu ermöglichen.

21. Verfahren nach einem der Ansprüche 18 bis 20, wobei das Glycosaminoglycan Heparin ist und das Protein Antithrombin IIII (AT III) ist.

22. Verfahren zur Herstellung eines kovalenten Konjugats nach einem der Ansprüche 1 bis 17 mit den Stufen der Konjugierung eines Proteins, welches wenigstens eine primäre Aminogruppe umfaßt, mit einem Glycosaminoglycan durch ein Verfahren nach einem der Ansprüche 18 bis 21.

23. Verfahren nach Anspruch 24, welches auch die Stufe der Isolierung des kovalenten Konjugats umfaßt.

24. Verfahren zur Herstellung einer Konjugatzusammensetzung von Proteinen, die wenigstens eine durch kovalente Bindungen mit Glycosaminoglycanen verbundene primäre Aminogruppe umfassen, wobei man bei dem Verfahren:
(a) die Glycosaminoglycane mit den Proteinen bei einem pH-Wert und über einen Zeitraum, der für die Ausbildung eines Imins zwischen der Aminogruppe und einem terminalen Aldoserest der Glycosaminoglycane ausreichend ist, inkubiert, und über eine Zeit und bei einer Temperatur, die dafür ausreicht, daß die Imine eine anschließende Amadori-Umlagerung über einen ausreichenden Zeitraum in ein α-Carbonylamin unter Ausbildung der kovalenten Bindungen durchlaufen, und
(b) die Konjugatzusammensetzung oder ein pharmazeutisch verträgliches Salz davon isoliert, wobei die Glycosaminoglycane Heparin (H) sind und die Amino-enthaltenden Moleküle Antithrombin III (AT III) sind.

25. Verfahren nach Anspruch 20 oder 24, wobei das Inkubieren für etwa 3 Tage bis 2 Wochen bei einer Temperatur von 35°C bis 45°C durchgeführt wird.

26. Verfahren nach Anspruch 25, wobei das Inkubieren für etwa 2 Wochen durchgeführt wird.

27. Pharmazeutisches Präparat, welches ein Konjugat gemäß einem der Ansprüche 1 bis 17 als aktiven Bestandteil in Verbindung mit einem pharmazeutisch verträglichen Träger enthält.

28. Pharmazeutisches Präparat nach Anspruch 27 in der Form einer wäßrigen Lösung zur Injektion oder in der Form einer Salbe oder in der Form eines Aerosols.

29. Verwendung eines Konjugats nach einem der Ansprüche 1 bis 17 zur Herstellung eines Arzneimittels für die prophylaktische und therapeutische Behandlung des Atemnotsyndroms von Neugeborenen, des Atemnotsyndroms von Erwachsenen, des Primärkarzinoms der Lunge, des non-Hodgkins-Lymphoms, der fibrosierenden Alveolitis, von Lungentransplantaten, der L-Asparaginase-induzierten Defizienz, der Herz-Lungen-Umgehungs-induzierten Defizienz, von Sepsis oder vererbbaren AT-III-Mangelzuständen.

30. Verwendung nach Anspruch 29, wobei das Säugetier ein Mensch ist.

31. Verwendung eines Konjugats nach einem der Ansprüche 1 bis 17 zur Herstellung eines Arzneimittels zur Vorbeugung und Behandlung von Thrombose.

32. Verwendung eines Konjugats nach einem der Ansprüche 1 bis 17 zur Herstellung eines Arzneimittels für die Behandlung des Atemnotsyndroms eines Kindes oder Erwachsenen.

33. Verwendung nach Anspruch 32, wobei das Konjugat nach Anspruch 9 unmittelbar den Atemwegen der Lunge zugeführt wird.

34. Verwendung nach Anspruch 33, wobei gleichzeitig ein Lungenoberflächenfaktor, ein anti-inflammatorisches Steroid, ein anti-Asthma-Arzneimittel oder ein bronchienerweiterndes Mittel verabreicht wird.

35. Verwendung eines Konjugats nach einem der Ansprüche 1 bis 17 zur Herstellung eines Arzneimittels zur Verhinderung der Fibrinabscheidung in den Lungenalveolen.

36. Material zur Verwendung in einer medizinischen oder prosthetischen Vorrichtung, wobei das Material ein Polymer umfaßt, welches Kontakt mit einem kovalenten Konjugat nach einem der Ansprüche 1 bis 17 hat.

37. Material nach Anspruch 36, wobei das Glycosaminoglycan Heparin ist und die Spezies Antithrombin III ist.

38. Material nach Anspruch 36 oder 37, wobei das Konjugat an das Polymer kovalent angeheftet ist.

39. Material zur Verwendung in einer medizinischen oder prosthetischen Vorrichtung, wobei das Material ein Polymer umfaßt, das mit einer kovalenten Konjugatzusammensetzung Kontakt hat, wobei die kovalente Konjugatzusammensetzung Glycosaminoglycane umfaßt, die über kovalente Verknüpfungen mit einem Protein verknüpft sind, wobei das Protein wenigstens eine primäre Aminogruppe umfaßt, wobei die Glycosaminoglycane Heparine sind, das Protein Antithrombin III ist und die kovalente Konjugatzusammensetzung Antithrombin-III-Heparin (ATH) umfaßt, wobei das ATH kovalent an das Polymer angeheftet ist, und wobei das Protein über die Aminogruppe mit einem terminalen Aldoserest des Glycosaminoglycans mittels einer α-Carbonylaminverknüpfung unmittelbar kovalent verknüpft ist.

40. Material nach einem der Ansprüche 36 bis 39, wobei das Polymer ein synthetisches Polymer ist, das ausgewählt ist unter Poly-2-hydroxyethylmethoacrylat, Polyacrylamid, Polyether-Polyurethanharnstoff (PEUU), Polyethylen, Polypropylen, Polytetrafluorethylen, Poly-(vinylchlorid), Polydimethylsiloxan, einem Ethylen-Acrylsäure-Copolymer, Dacron, PolyesterPolyurethan, Polyurethan, Polycarbonat-Polyurethan, Polyamid (Nylon) und Polystyren.

41. Material nach Anspruch 40, wobei das Polymer Polyurethan-Polycarbonat ist.

42. Prosthetische oder medizinische Vorrichtung, welche ein Material nach einem der Ansprüche 36 bis 41 umfaßt.

43. Vorrichtung nach Anspruch 42, die ausgewählt ist unter endovaskulären Schläuchen, einer arteriellen oder zentralvenösen Leitung, einem Herzkatheter, einem Herz-Lungen-Umgehungskreislauf, einem Dialysekreislauf oder einem anderen mit externem Blut in Kontakt tretenden Instrument, einer Schrittmacherableitung, einem arteriellen oder venösen Katheter, einem Thrombektomiekatheter, einer Sutur, einem Blutfilter, einer intravenösen Leitung, einem mechanischen Ventil, einem Stent, einer/m künstlichen Niere, Lunge, Herzen oder Leber und einer in-vivo-Prothese.

44. Vorrichtung nach Anspruch 43, wobei die Vorrichtung ein endovaskulärer Schlauch ist.

45. Verfahren zur Verringerung der Thrombenbildungsfähigkeit eines Materials, bei dem man das Material mit einem Konjugat nach einem der Ansprüche 9 bis 13 beschichtet.

46. Verfahren nach Anspruch 45, wobei das Konjugat kovalent mit dem Material verbunden ist.

47. Verfahren nach Anspruch 45 oder 46, wobei das Material für die Verwendung in einer prosthetischen oder medizinischen Vorrichtung nach Anspruch 43 geeignet ist.

48. Verwendung eines Konjugats nach einem der Ansprüche 1 bis 17 zur Verringerung der Thrombenbildungsfähigkeit von inneren oder äußeren Vorrichtungen, die mit Blut in Kontakt treten.

## Revendications

1. Conjugué covalent comprenant un glycosaminoglycanne lié à une protéine par une liaison covalente, dans lequel ladite protéine comprend au moins un groupe amino primaire, ladite protéine est liée directement par covalence par ledit groupe amino à un résidu aldose terminal dudit glycosaminoglycanne, ladite liaison covalente comprenant une α-carbonylamine formée par un réarrangement d'Amadori ultérieur, pendant une période de temps suffisante, d'une imine résultant de la réaction entre ledit groupe amino et ledit résidu aldose terminal dudit glycosaminoglycanne et ses sels pharmaceutiquement acceptables, ledit glycosaminoglycanne étant l'héparine.

2. Conjugué suivant la revendication 1, dans lequel ladite liaison covalente comprend un groupe -CO-CH₂-NH- formé par réarrangement d'Amadori d'un groupe -HCOH-HC=N-résultant de la réaction entre ledit groupe amino et le groupe carbonyle C1 dudit résidu aldose terminal.

3. Conjugué suivant la revendication 2, dans lequel, dans le groupe -CO-CH₂-NH-, la portion -CO-CH₂ est dérivée du glycosaminoglycanne et la portion -NH- est dérivée du groupe amino de la protéine.

4. Conjugué qui répond à la formule : glycosaminoglycanne-CO-CH₂-NH-protéine, dans lequel le glycosaminoglycanne est l'héparine et la protéine est l'antithrombine III (AT).

5. Conjugué suivant l'une quelconque des revendications précédentes, dans lequel le rapport molaire de ladite protéine au glycosaminoglycanne est inférieur à un.

6. Conjugué suivant l'une quelconque des revendications 1 à 5, dans lequel le rapport molaire de ladite protéine au glycosaminoglycanne est de 1:1.

7. Conjugué suivant l'une quelconque des revendications précédentes, dans lequel ladite protéine est un inhibiteur de sérine-protéase.

8. Conjugué suivant la revendication 7, dans lequel ledit inhibiteur de protéase est choisi dans le groupe consistant en l'antithrombine III et le cofacteur II d'héparine.

9. Conjugué suivant l'une quelconque des revendications précédentes, dans lequel le glycosaminoglycanne est l'héparine et la protéine est l'antithrombine III.

10. Conjugué suivant la revendication 9, qui est plus efficace pour inhiber la thrombine que l'antithrombine III et l'héparine libres.

11. Conjugué suivant la revendication 9, ayant un poids moléculaire de 69kD à 100kD.

12. Conjugué suivant la revendication 9, 10 ou 11, qui possède plus de 60 %, 90 %, 95 % ou 98 % de l'activité d'antithrombine de l'héparine non conjuguée intacte.

13. Conjugué suivant la revendication 9, qui a une demi-vie supérieure à celle de l'héparine *in vivo*.

14. Conjugué suivant l'une quelconque des revendications précédentes, dans lequel le glycosaminoglycanne est l'héparine et l'héparine comprend un pentasaccharide unique.

15. Conjugué suivant l'une quelconque des revendications précédentes, dans lequel le glycosaminoglycanne est l'héparine et l'héparine comprend deux pentasaccharides.

16. Conjugué suivant la revendication 15, comprenant plus de 10 % de chaînes d'héparine comportant deux pentasaccharides.

17. Conjugué suivant l'une quelconque des revendications précédentes, pharmaceutiquement apte à l'injection à un être humain.

18. Procédé en une étape pour la conjugaison d'une protéine comprenant au moins un groupe amino primaire à un glycosaminoglycanne, ledit procédé comprenant la mise en incubation dudit glycosaminoglycanne avec ladite protéine dans des conditions qui permettent la formation d'une imine entre ledit groupe amino et un résidu aldose terminal dudit glycosaminoglycanne, ledit glycosaminoglycanne étant l'héparine, et l'imine étant réarrangée en une α-carbonylamine.

19. Procédé suivant la revendication 18, dans lequel ladite α-carbonylamine est formée par réarrangement d'Amadori de l'imine.

20. Procédé suivant la revendication 18, qui comprend la mise en incubation dudit glycosaminoglycanne avec ladite protéine à un pH convenable pour la formation d'imine entre ledit groupe amino et le résidu aldose terminal du glycosaminoglycanne et pendant une période de temps suffisante pour permettre un réarrangement d'Amadori de ladite imine en une α-carbonylamine.

21. Procédé suivant l'une quelconque des revendications 18 à 20, dans lequel le glycosaminoglycanne est l'héparine et la protéine est l'antithrombine III (AT III).

22. Procédé pour la production d'un conjugué covalent suivant l'une quelconque des revendications 1 à 17, qui comprend l'étape de conjugaison d'une protéine comprenant au moins un groupe amino primaire à un glycosaminoglycanne par un procédé suivant l'une quelconque des revendications 18 à 21.

23. Procédé suivant la revendication 24, qui comprend également l'étape d'isolement du conjugué covalent.

24. Procédé pour la préparation d'une composition conjuguée de protéines comprenant au moins un groupe amino primaire liées à des glycosaminoglycannes par des liaisons covalentes, ledit procédé comprenant :
(a) la mise en incubation desdits glycosaminoglycannes avec lesdites protéines à un pH et pendant une période de temps suffisants pour la formation d'une imine entre ledit groupe amino et un résidu aldose terminal desdits glycosaminoglycannes, et en une période de temps et à une température suffisantes pour que lesdites imines subissent un réarrangement d'Amadori ultérieur, pendant un période de temps suffisante, en une α-carbonylamine formant lesdites liaisons covalentes ; et
(b) l'isolement de ladite composition conjuguée ou d'un de ses sels pharmaceutiquement acceptables, où lesdits glycosaminoglycannes consistent en héparine (H) et lesdites molécules contenant des groupes amino consistent en antithrombine III (ATIII).

25. Procédé suivant la revendication 20 ou 24, dans lequel ladite incubation est effectuée pendant une période d'environ 3 jours à deux semaines à une température de 35°C à 45°C.

26. Procédé suivant la revendication 25, dans lequel ladite incubation est effectuée pendant environ deux semaines.

27. Préparation pharmaceutique contenant un conjugué suivant l'une quelconque des revendications 1 à 17 comme ingrédient actif en association avec un support pharmaceutiquement acceptable.

28. Préparation pharmaceutique suivant la revendication 27, sous forme d'une solution aqueuse pour injection ou sous forme d'une pommade ou sous forme d'un aérosol.

29. Utilisation d'un conjugué suivant l'une quelconque des revendications 1 à 17 dans la production d'un médicament destiné au traitement prophylactique et thérapeutique du syndrome de détresse respiratoire du nouveau-né, du syndrome de détresse respiratoire de l'adulte, d'un carcinome primaire du poumon, d'un lymphome non hodgkinien, de l'alvéolite fibrosante, de transplants pulmonaires, de la déficience induite par la L-asparaginase, de la déficience induite par une dérivation cardio-pulmonaire, de la septicémie ou d'états de déficience congénitale en ATIII.

30. Utilisation suivant la revendication 29, dans laquelle ledit mammifère est un être humain.

31. Utilisation d'un conjugué suivant l'une quelconque des revendications 1 à 17, dans la production d'un médicament destiné à la prévention et au traitement de la thrombose.

32. Utilisation d'un conjugué suivant l'une quelconque des revendications 1 à 17, dans la production d'un médicament destiné au traitement du syndrome de détresse respiratoire du nourrisson ou de l'adulte.

33. Utilisation suivant la revendication 32, dans laquelle le conjugué de la revendication 9 est délivré directement aux voies aériennes du poumon.

34. Utilisation suivant la revendication 33, dans laquelle un agent tensioactif pulmonaire, un stéroïde anti-inflammatoire, un médicament antiasthmatique ou un bronchodilatateur est administré conjointement.

35. Utilisation d'un conjugué suivant l'une quelconque des revendications 1 à 17 dans la production d'un médicament destiné à la prévention du dépôt de fibrine dans les alvéoles du poumon.

36. Substance destinée à être utilisée dans un dispositif médical ou prothétique, ladite substance comprenant un polymère en contact avec un conjugué covalent suivant l'une quelconque des revendications 1 à 17.

37. Substance suivant la revendication 36, dans laquelle le glycosaminoglycanne est l'héparine et l'entité est l'antithrombine III.

38. Substance suivant la revendication 36 ou 37, dans laquelle le conjugué est fixé par covalence au polymère.

39. Substance destinée à être utilisée dans un dispositif médical ou prothétique, ladite substance comprenant un polymère en contact avec une composition conjuguée covalente, ladite composition conjuguée covalente comprenant des glycosaminoglycannes liés à une protéine par des liaisons covalentes, la protéine comprenant au moins un groupe amino primaire, les glycosaminoglycannes étant des héparines, la protéine étant l'antithrombine III, et la composition conjuguée covalente comprenant de l'antithrombine III-héparine (ATH), ladite ATH étant fixée par covalence au polymère, et la protéine étant liée directement par covalence par ledit groupe amino à un résidu aldose terminal des glycosaminoglycannes par une liaison α-carbonylamine.

40. Substance suivant l'une quelconque des revendications 36 à 39, dans laquelle le polymère est un polymère synthétique choisi dans le groupe consistant en le poly(méthacrylate de 2-hydroxyéthyle), le polyacrylamide, la polyéther-polyuréthanne-urée (PEUU), le polyéthylène, le polypropylène, le polytétrafluoréthylène, le poly(chlorure de vinyle), le polydiméthylsiloxane, un copolymère éthylène-acide acrylique, le Dacron, un polyester-polyuréthanne, un polyuréthanne, un polycarbonate-polyuréthanne, un polyamide (Nylon) et un polystyrène.

41. Substance suivant la revendication 40, dans laquelle le polymère est un polyuréthanne-polycarbonate.

42. Dispositif prothétique ou médical comprenant la substance de l'une quelconque des revendications 36 à 41.

43. Dispositif suivant la revendication 42, choisi dans le groupe consistant en une tubulure endovasculaire, un conduit artériel ou conduit veineux central, un cathéter cardiaque, un circuit de dérivation cardio-pulmonaire, un circuit de dialyse ou un autre instrument externe en contact avec le sang, un fil de stimulateur cardiaque, un cathéter artériel ou veineux, un cathéter de thrombectomie, une suture, un filtre de sang, un conduit intraveineux, une valve mécanique, un expanseur, un rein, un poumon, un coeur ou un foie artificiel et une prothèse *in vivo.*

44. Dispositif suivant la revendication 43, le dispositif étant une tubulure endovasculaire.

45. Procédé pour réduire la thrombogénicité d'une matière, comprenant le revêtement de cette matière avec un conjugué suivant l'une quelconque des revendications 9 à 13.

46. Procédé suivant la revendication 45, dans lequel le conjugué est lié par covalence à la matière.

47. Procédé suivant la revendication 45 ou 46, dans lequel la matière est apte à l'utilisation dans un dispositif prothétique ou médical, comme défini dans la revendication 43.

48. Utilisation d'un conjugué suivant l'une quelconque des revendications 1 à 17 pour réduire la thrombogénicité de dispositifs internes et extracorporels qui entrent en contact avec le sang.
